# EUROPEAN PATENT APPLICATION

(11) **EP 2 530 163 A1**
(43) Date of publication of application: **05.12.2012**
(21) Application number: 11168153.2
(22) Date of filing: 31.05.2011
(51) Int. Cl.: C12N 15/90, C12N 15/85, C12N 5/0735

(54) **Double marker cell line**

(71) Applicant: Veterinärmedizinische Universität Wien, 1210 Vienna (AT)
(72) Inventor: Kautschitsch, Susanna, 1030 Wien (AT)
(74) Representative: Sonn & Partner Patentanwälte

(57) **Abstract**

The invention describes a method for producing a knock in targeted mutation in cells by using a double marker cell line. The double marker cell line genotype is heterozygous, wherein on one allele a promoter is operably linked to a polynucleotide sequence encoding a marker protein and on the other allele a promoter is linked to a polynucleotide sequence encoding another marker protein is present; and obtaining a double marker cell line, especially a double marker embryonic stem (ES) cell line, from this genotype.

SEQUENCE LISTING

<110>  VETERINÄRMEDIZINISCHE UNIVERSITÄT WIEN

<120>  Gene Targeting

<130>  R 57018

<160>  10

<170>  PatentIn version 3.5

<210>  1
<211>  21
<212>  DNA
<213>  artificial

<220>
<223>  artificial

<400>  1
cctggaaaat gcttctgtcc g                                                21


<210>  2
<211>  21
<212>  DNA
<213>  artificial

<220>
<223>  artificial

<400>  2
cagggtgtta taagcaatcc c                                                21


<210>  3
<211>  21
<212>  DNA
<213>  artificial

<220>
<223>  artificial

<400>  3
ctctgctgcc tcctggcttc t                                                21


<210>  4
<211>  21
<212>  DNA
<213>  artificial

<220>
<223>  artificial

<400>  4
cgaggcggat cacaagcaat a                                                21


<210>  5
<211>  19
<212>  DNA
<213>  artificial

<220>
<223>  artificial

<400>  5
tcaatgggcg ggggtcgtt                                                   19

<210>  6
<211>  19
<212>  DNA
<213>  artificial

<220>
<223>  artificial

<400>  6
gagcacagga acaattggc                                                    19


<210>  7
<211>  19
<212>  DNA
<213>  artificial

<220>
<223>  artificial

<400>  7
agacattacc ttcgccacc                                                    19


<210>  8
<211>  550
<212>  DNA
<213>  artificial

<220>
<223>  artificial

<400>  8
agacattacc ttcgccaccc ccacctcaca ttcttggact ccctgtggcg tatgccccag      60

tatccttgcg atatccctgt tgaatttgta gggttctagc ctggaattga gccagatttt     120

gtcgttttcc ctgcctaggg cgtggagagc gatcttttaa gaacgactct aaagaagagg     180

gggagggata tgcccctatg aatccgtgca agccaagatc ctaggtaaat ggtagtgggc     240

aaaagccctg gttttagtag gtaatcgctc agaaggaaag ttttaactcc tgggaaagcc     300

ccttctatgc taatttattc tatcgggcgc ctttgccttt ctcattctta ggaataagga     360

tctttctcct gtgtactgat gctgcagccc gccctgctgg cctgggtcag gggtgcgtgg     420

atgacctgca ttttctgctt tctcatgtta cttgtgtaac gtcttcacca gcaactcatt     480

tttcgcccag agctctgggc tctgggtttt gtctatacct ggattccttt tgccaattgt     540

tcctgtgctc                                                            550


<210>  9
<211>  20
<212>  DNA
<213>  artificial

<220>
<223>  PCR molecule/probe


<220>
<221>  misc_feature
<222>  (1)..(20)

<400>  9

cctcgacctg cagcccaagc                                                    20

<210>  10
<211>  23
<212>  DNA
<213>  artificial

<220>
<223>  PCR molecule/probe

<220>
<221>  misc_feature
<222>  (1)..(23)

<400>  10
gcagtgttga gggcaatctg gga                                                23

## Description

The present invention concerns cell lines with markers, especially fluorescent embryonic stem- (ES-) cell lines, the generation thereof and animals comprising such (ES) cells.

The introduction of targeted mutations in mouse embryonic stem (ES) cells is a valuable tool to study gene function in mammals. However, the identification of targeted ES cell clones is still a laborious and time consuming procedure. Enrichment for the rare targeting events is usually achieved by incorporating selection marker into the targeting vector and specific treatment of the cultured cells to eliminate the majority of non-mutated clones. Further analysis via PCR and Southern blot are indispensable to identify and verify homologous recombination (HR).

Thomas et al. (Cell 51 (1987), 503-512) mutated, by gene targeting, the endogenous hypoxanthine phosphoribosyl transferase (HPRT) gene in mouse embryo-derived stem (ES) cells. This protocol is useful for targeting mutations into any gene. Such first generation chimeras (generated by injection of the genetically modified ES cells into a blastocyst) are usually heterozygous for the targeted mutation. Subsequent breeding can be used to generate the homozygous animal. This technology was proposed to dissect the developmental pathway of the mouse as well as to generate mouse models for human genetic diseases. ES cells have been shown to be pluripotent in vitro and in vivo. When introduced into mouse blastocysts, these cells contribute efficiently to the formation of chimeras, including contributions to a functional germ line. In addition, these cells can be manipulated in vitro without losing their capacity to generate germ-line chimeras (Thomas et al., 1987).

Accordingly, there is a need for a new approach for rapid identification of homologous integration events in ES cells. Such new approach should allow easy and reliable integration of genes of interest or mutations. Expression of the gene of interest or the mutated gene in ES cells can be immediate or conditional. Moreover, immediate and uncomplicated information should be available whether such a gene of interest, or a mutation, to be expressed, has been properly introduced in the genome of the ES cells and at which position such integration in the genome has occurred.

Therefore, the present invention provides a method for producing a double marker cell line comprising the following steps:
- providing a first genotype containing a first polynucleotide comprising
   (a) a promoter operably linked to
   (b) a first polynucleotide sequence encoding a first marker protein heterologous to the first genotype,
   (c) said first polynucleotide sequence encoding a first marker protein being flanked by site specific recombination sites ("SSRS"), a 5' SSRS and a 3' SSRS; and
   (d) a second polynucleotide sequence encoding a second marker protein heterologous to the first cell line, being located 3' to the 3' SSRS site;
- providing a second genotype capable of expressing a recombinase being specific for said SSRSs;
- crossing said first genotype with said second genotype wherein said recombinase deletes the first polynucleotide sequence encoding a first marker protein so as to obtain a third genotype wherein said promoter is operably linked to said second polynucleotide sequence encoding said second marker protein and wherein the recombinase gene is still present in said third genotype;
- crossing said third genotype with a fourth genotype capable of expressing a recombinase being specific for said SSRSs thereby obtaining a fifth genotype wherein said promoter is operably linked to said second polynucleotide sequence encoding said second marker protein and wherein the recombinase gene is not present in said fifth genotype;
- crossing said fifth genotype with a sixth genotype, said sixth genotype containing a third polynucleotide comprising
   (a) a promoter operably linked to
   (b) a third polynucleotide sequence encoding a third marker protein heterologous to the first genotype,
   (c) said third polynucleotide sequence encoding a third marker protein being flanked by SSRSs, a 5' SSRS and a 3' SSRS; and
   (d) said second polynucleotide sequence encoding said second marker protein heterologous to the first genotype, being located 3' to the 3' SSRS;
thereby obtaining a seventh genotype which is heterozygous and wherein on one allele said promoter is operably linked to said second polynucleotide sequence encoding said second marker protein and on the other allele said third polynucleotide is present; and
- obtaining a double marker cell line, especially a double marker embryonic stem (ES) cell line, from said seventh genotype.

This method according to the present invention provides a new approach for rapid identification of homologous integration events in cells, cell lines of animals (or other eukaryotic or prokaryotic genotypes), here especially in ES cells. According to the present invention, a cell line with two markers is provided. For example, a double fluorescent ES cell line descending from the double fluorescent Cre reporter mouse Gt(ROSA)26Sortm4(ACTB-tdTomato,-EGFP)Luo/J (Muzumdar et al., 2007) was established. The novel (ES) cell line according to the present invention enables a less time consuming gene targeting procedure for knock-in applications into defined and predetermined locations in the chromosome of the ES cell, e.g. into the Rosa26 locus, by simply visualizing targeting events by the lack of fluorescence expression in vitro (the Rosa26 locus of the mouse genome is commonly used as a knock-in targeting system to ubiquitously or conditionally express cDNAs). The novel approach according to the present invention facilitates this knock-in procedure by allowing the identification and selection of positive (ES-)cell clones based on their marker signal, preferably based on a marker fluorescence signal. The presented invention provides a new (ES) cell line system which allows for fast and reliable identification of targeted clones.

The cell line according to the present invention which can be used for receiving a foreign gene of interest (which should be expressed) into its genome (the "seventh cell line") is characterised by a heterozygotic nature: on one allele said promoter is operably linked to said second polynucleotide sequence encoding said second marker protein (preferably a fluorescence marker) and on the other allele said third polynucleotide encoding a different marker protein (preferably another fluorescence marker) is present (it is, of course, self-understanding that the two different markers must be distinguishable from each other by at least one feature, preferably, they differ in colour of the expressed gene product of the marker gene. If, therefore, the gene of interest is introduced e.g. by electroporation, the targeting construct ("eighth genotype") is - in contrast to the other genotypes described herein - a DNA construct but not a cell line or animal. The targeting construct carries the gene of interest or the mutation between the homologous arms and either integrates at the first allele or at the second allele or in rare cases on both alleles. The resulting cell line then reports the success of the targeted integration of the targeting construct by the lack of one or both fluorescence signals. The lack of one fluorescence signal indicates the successful formation of the ninth cell line and gives information at which allele the recombination has taken place and the site of recombination (i.e. where the recombination site is foreseen, e.g. at the Rosa26 locus).

The present invention also relates to a method for producing a genotype capable of expressing a gene of interest (ninth genotype), said gene of interest being heterologous for said genotype characterised in that an eighth genotype is introduced into the double marker ES cell line according to the seventh genotype,
- said eighth genotype containing a fourth polynucleotide comprising a gene of interest encoding a gene to be expressed in said ninth genotype and being flanked by DNA sequences which are homologous to the 5' and 3' regions from the marker genes in said seventh genotype so as to allow homologous recombination between at least one allele of the seventh genotype and said eighth genotype;
   said ninth genotype being heterozygous and comprising either
   (a) on one allele said second polynucleotide sequence encoding said second marker protein, being located 3' to the 3' SSRS, and on the other allele said fourth polynucleotide being flanked by SSRSs, a 5' SSRS and a 3' SSRS, is present ("ninth genotype A"); or
   (b) on one allele said third polynucleotide sequence encoding said third marker protein being flanked by SSRS, a 5' SSRS and a 3' SSRS and on the other allele said fourth polynucleotide being flanked by SSRSs, a 5' SSRS and a 3' SSRS, is present ("ninth genotype B"); said ninth genotype therefore being either (a) capable of expressing said gene of interest together with said second marker ("ninth genotype A") or (b) capable of expressing said gene of interest together with said third marker ("ninth genotype B").

According to the general strategy to obtain the genotypes according to the present invention, i.e. especially the seventh and the ninth genotype, the third marker can be identical to the first marker (of course, also identical promoters can be used). In a preferred embodiment, the first genotype is therefore similar or identical with the sixth genotype.

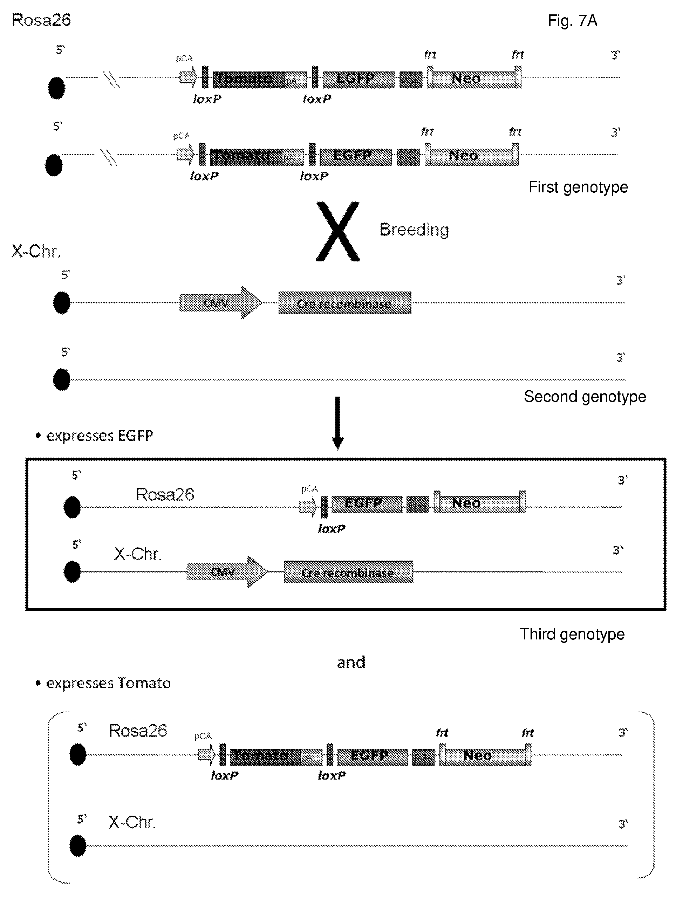

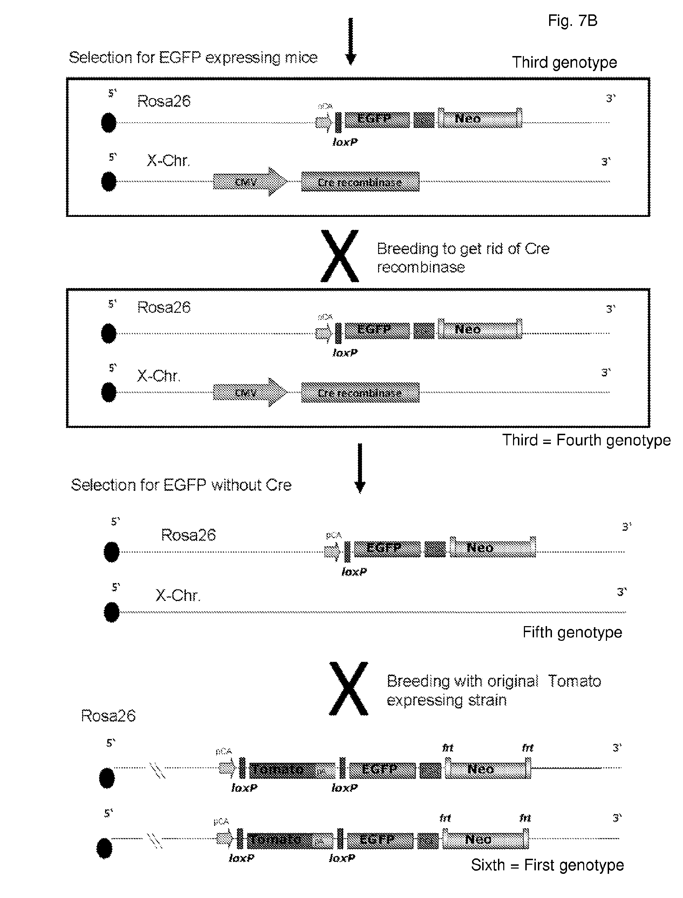

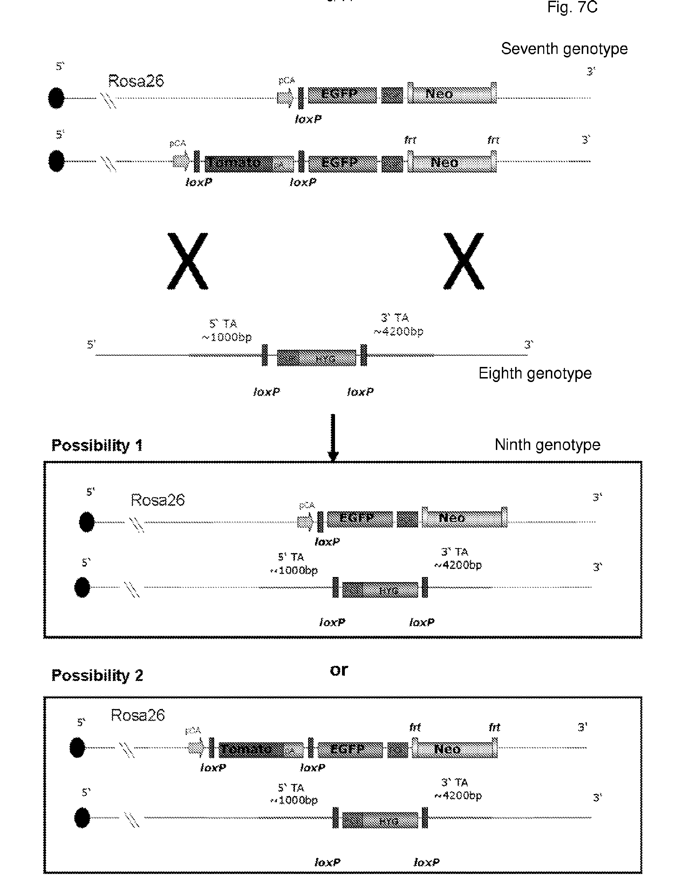

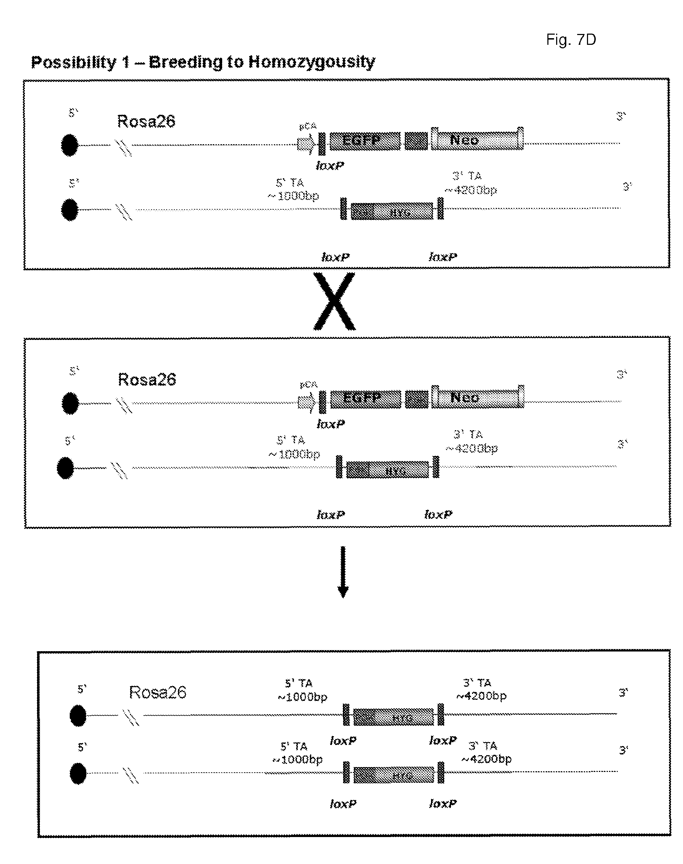

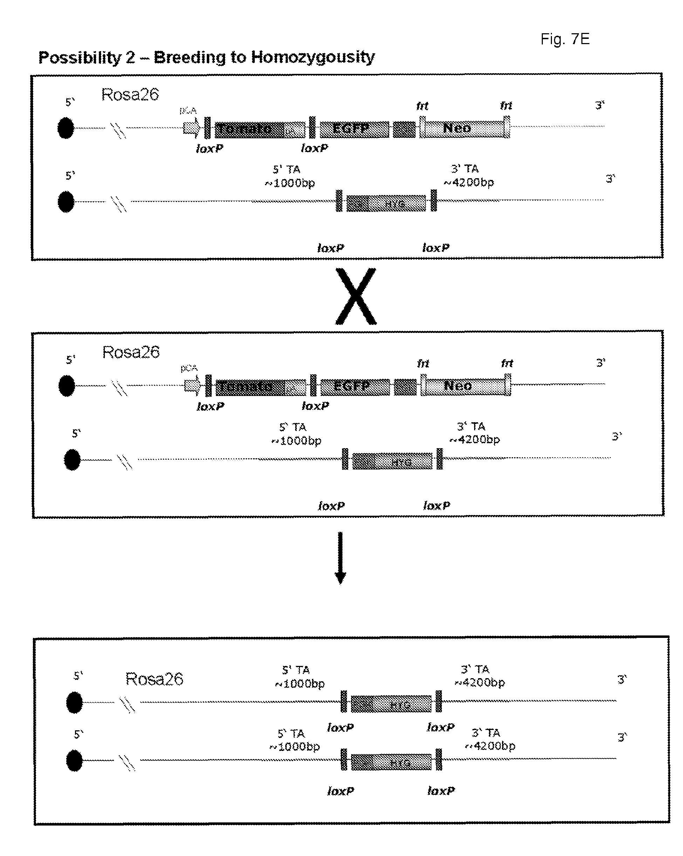

The DNA sequences flanking the gene of interest are homologous to the 5' and 3' regions from the marker genes in said seventh genotype so as to allow homologous recombination between at least one allele of the seventh genotype and said eighth genotype. These DNA sequences allowing homologous recombination are depicted as red lines in the genotypes depicted in Fig. 7.

A specifically preferred embodiment of the first and/or the sixth genotype is the genotype (cell line) disclosed by Muzumdar et al. (Genesis 45 (2007), 593-605; in contrast the other genotypes generated by the same research group for other purposes (Zong et al., Cell 121 (2005), 479-492; US patent No. 7,282,621 B2 (describing the "mosaic analysis with double markers" (MADM) method))). This genotype comprises a double-fluorescent Cre reporter mouse that expresses membrane-targeted tandem dimer Tomato (mT) prior to Cre-mediated excision and membrane-targeted green fluorescent protein (mG) after excision. It was shown that reporter expression is nearly ubiquitous, allowing visualization of fluorescent markers in live and fixed samples of all tissues examined. In this cell line, it was further demonstrated that mG labeling is Cre-dependent, complementary to mT at single cell resolution, and distinguishable by fluorescence-activated cell sorting. Both membrane-targeted markers outline cell morphology, highlight membrane structures, and permit visualization of fine cellular processes.

However, quite in contrast, this (mouse) genotype is used within the course of the present invention to generate a heterozygous double (fluorescent) marker cell line which can be used for introduction of foreign genes of interest in a reproducible manner and with a straightforward information capacity for the integration event. For example, the original constructs for the MADM method do not show fluorescence expression at all before Cre recombination whereas the constructs according to the present invention allow expression of two reciprocal marker (e.g. fluorescence) genes which cause expression of the marker (e.g. fluorescence) genes at all time. The MADM constructs allow fluorescence gene expression only after (Cre) recombination and fusion of each N-terminal and C-terminal marker genes. According to the present invention, the marker gene is replaced by the gene of interest which is located within the homologous arms of the targeting construct. The MADM system, in contrast, has its gene of interest located 5' from the N-GFP and is therefore not replacing a marker gene, but rather adding it to the present fluorescence genes. Moreover, MADM methods require a Cre recombinase encoded in the genome whereas such Cre recombinase is not required for the present invention to be encoded in the genome of the genotype. The genotype described in Muzumdar et.al. 2007 presents the genome used to establish the double fluorescent ES cell line present in this invention. Zong et. al. 2005 and US patent No. 7,282,621 B2 describe the MADM System, which is not representing any step nor any strategy of the present invention. The genotype described in Muzumdar et.al., which was constructed for a different purpose, is suitable as a possible starting and intermediate product for obtaining the genotypes according to the present invention.

A "genotype" according to the present invention is the genetic makeup of a nucleotide (preferably DNA) construct, a cell, a cell line, an organism (e.g. an animal), or an individual (i.e. the specific allele makeup of the individual). The "first", "second", etc. genotype refer to genetic properties of a given genotype which has the specific character mentioned and explained above and hereinbelow. The numbering by itself, however, is not meant to exactly correspond to the sequence of steps in a process. More specifically, the numbering "fifth genotype" would not necessarily indicate by itself that a first to fourth genotype are necessary in carrying out the described method. This holds true similarly for the numbering given to cell lines polynucleotide sequences, markers, and the like.

If possible, the "genotype" of a certain crossing step above refers to all practically possible embodiments of this genotype. Preferably, the genotype is crossed as individual animal ("breeding"). According to a preferred embodiment, the present invention is performed with rodent or lagomorpha genotypes, especially rat, mouse, rabbit or guinea pig animals. These animals are established laboratory animals and are easily crossable (breedable) in a laboratory environment by established breeding methods (especially mouse or rat animals). Specifically for mouse crossing and breeding steps, a vast number of manuals exist for conducting such steps, such as Nagy et al, "Manipulating the Mouse Embryo" 3rd Ed. (2003), CSH Laboratory Press, Cold Spring Harbour, NY. Other key technology papers of specific use for the methods described herein include Buehr et al., Cell 135 (2008) 1287-1298; Irion et al., Nat. Biotechnol. 25 (2007), 1477-1482; Kawamata et al., Methods Mol. Biol. 597 (2010), 169-177; Li et al., Cell 135 (2008), 1299-1310; Soriano et al., Nat. Genet. 21 (1999), 70-71; Srinivas et al., BMC. Dev. Biol., 1 (2001) 4; Tong et al., Nature, 467 (2010) 211-213; and Zwaka et al., Nat. Biotechnol., 21 (2003) 319-321.

The present invention optionally excludes methods according to Art. 53(a) EPC. Further optionally, the invention excludes the use of a human embryo for industrial or commercial purposes. Still further optionally, the invention is not directed at the human body at the various stages of formation and development. Accordingly, where human ES cells are contemplated in the context of the invention, they are optionally not derived from industrial or commercial use of a human embryo, but from alternative sources (such as research use, medical intervention, or reprogramming of somatic cells and the like mtheods known to a skilled person). The ES cell lines according to the present invention are preferably pluripotent and will preferably not be capable of developing into an animal by themselves; this has to be specifically emphasised for human ES cell lines obtainable by the present invention.

The term "capable of expression" of a given gene means that the genotype either expresses the gene product of this gene or can - by way of methods known to a person skilled in the art of biotechnology - be induced to be expressed in a reproducible and controlled manner.

The result directly obtained from the method of the present invention where a heterologous gene of interest is present in the two possibilities of the ninth genotype is a heterozygous genotype. It is in many cases preferred to use a homozygous genotype for expressing the gene of interest. Accordingly, the heterozygous ninth genotype can easily made homozygous by further crossing or targeting steps thereby obtaining a homozygous ninth genotype. As shown in the example section for the ninth genotype, this has been achieved in this example in the following manner: the targeted ES cells have been injected into wildtype blastocysts. Resulting Embryos were chimeric (a mix between targeted red or green cells and wildtype cells). These animals (reared from these embryos) were bred to wildtype animals and where the germline compatible targeted cells are inherited to offspring, resulted in the birth of heterozygous animals being either red (expressing the red marker protein) or green (expressing the green marker protein). These heterozygous animals were then bred among each other resulting in 25% animals, homozygous for the gene of interest.

As already mentioned, one major field of application of the present invention is animal genetics, especially mouse and rat genetics. According to a preferred embodiment, a non-human animal (preferably a mammal, especially a rat or a mouse) is produced from said ninth genotype, especially from said ES cell line.

It is essential for the present invention that the third and the second marker are distinguishable from each other (as are the first and the second); on this difference, the selection and performance of the present invention is built. However, the first and the third marker do not have to be distinguishable, indeed they can be the same. In a practical (and therefore preferred) embodiment of the present invention, the first and the third marker are identical. In this case, the same or almost the same constructs can be used in the first and in the sixth genotype. Also the whole genotype can be identical (i.e. first genotype is similar or identical to the sixth genotype).

There are a number of different SSRSs to choose for a person skilled in the art. Of course, the SSRSs have to be used in combination with the appropriate recombinase. Preferably, SSRSs are chosen in the present invention which are well established in the laboratory practice, such as Cre-loxP sites (Cre being the recombinase used in combination with the loxP SSRE), Flp-frt (Flp being the recombinase used in combination with the frt SSRE)sites and Dre-rox sites (Dre being the recombinase used in combination with the rox SSRE).

According to a preferred embodiment, any one of the first to fourth polynucleotide (or two, three or all four of them) may further comprise a gene encoding a selection marker, preferably under the control of an eukaryotic promoter, especially under the control of the murine Phosphoglucokinase gene (PGK) promoter. The PGK promoter is a very efficient constitutive promoter which is well functioning in ES cells. Preferably, the gene encoding this selection marker is flanked by SSRSs, said SSRSs being different from said SSRSs which flank said first, second or third marker, said gene encoding said selection marker is preferably flanked by Flp-recombinase target (frt) sites, a 5' frt site and a 3' frt (especially, if loxP sites are used for the markers; loxP and frt sites do not interfere with each other).

A preferred selection marker is an antibiotic resistance gene, preferably neomycin, hygromycin, puromycin, 6-thioguanine and zeocin resistance genes. In the targeting process, it is indispensable to take care that also the feeder cells (e.g. fibroblasts in ES cell culture) carry the appropriate selection (resistance) genes. The cell culture conditions can then easily be adapted to a given type of selection substance, e.g. the antibioticum.

Examples for appropriate selection genes include neomycin phosphotransferase or aminoglycoside 3'-phosphotransferase gene (Neo), hypoxanthine phosphoribosyltransferase (hprt), xanthin/guanin phosphoribosyltransferase (gp) or dihydrofolatreduktase (dhfr).

The nature of the genotype is in principle not critical, generally, the present invention can be applied in any species wherein the above mentioned prerequisites and crossing events can be established. Of course, it is practically preferred, if the genotypes are genotypes for which already a laboratory practice in gene technology exists. Accordingly, it is preferred that the genotypes and the (ES) cell lines according to the present invention are murine, rat, rabbit, jack rabbit, primate, preferably human, cat, dog, sheep, goat, bovine, pig, frog, fish, insect, plant, especially moss, tobacco, maize, Arabidopsis, rice or barley, equine or guinea pig genotypes and (ES) cell lines, especially murine or rat genotypes and (ES) cell lines. Preferred animals are mammals, especially rodents. Also human cells or cell lines are preferred genotypes in certain embodiments.

The markers according to the present invention should allow rapid information of the integration event of the gene of interest. Therefore, the second marker has to be distinguishable from the first and third marker. Therefore, any combination of at least two different markers can be used according to the present invention. Of course, all marker genes used according to the present invention are heterologous for the genotype in which they are introduced, i.e. they are not present in the wild type genotype of this cell line, preferably the ES cell line, if selection using the marker is to be carried out at the stage of the cell line, or the (strain of the) species, if selection using the marker is to be carried out at the level of the animal. The integration of a targeting construct at one of the two locations (preferably, on one of the two alleles) results in the loss of the respective marker signal, where the targeting construct is integrated. It is therefore sufficient that the two different markers can be properly expressed (e.g. by having a suitable promoter, a polyA sequence at the end of the gene, etc. If the constructs have integrated at the defined location in the genome, an integration event of the gene of interest is immediately evident by the loss of expression of either one or the other marker protein. It is also possible but probably rare, that both loci are targeted. Therefore both marker signals would get lost. This can therefore be optionally a third relevant - but rare - result of the ninth genotype. These genotype variants have no fluorescent markers expressed.

A preferred embodiment of the present invention is the use of fluorescent markers. Accordingly, at least one of said first, second or third markers may be fluorescence markers (preferably, all of them are fluorescence markers). Preferably, the markers are selected from a green or a red fluorescence protein, e.g. tandem dimer tomato (dtTomato), green fluorescent protein (GFP), enhanced green fluorescent protein (EGFP), red fluorescent protein (RFP), or another colour, such as enhanced blue fluorescent protein (EBFP), enhanced cyan fluorescent protein (ECFP), yellow fluorescent protein (YFP), or orange fluorescent protein.

Other fluorescence genes are well available for a person skilled in the art, the choice in each case should mainly depend on the distinguishability of the second marker from the first and the third marker and the functionality in the given genotype. For example, it is usually not appropriate to use two green markers as first and second marker even if they have (slightly) different wavelength specificities, because use of (significantly) different colours is more useful. It is also appropriate to use markers which do not have a significant impact on the viability of the genotype; markers which negatively affect the viability of the genotype should be prevented. Fluorescence genes to be included as a marker gene in the present invention include the fluorescence proteins such as mPlum, mKate, E2-Crimson, mRaspberry, HcRed1, HcRed-Tandem, mCherry, mStrawberry, AsRed2, DsRed, DsRed-Monomer, DsRed2, tdTomato (Tandem), dTomato, DsRed-Express2, DsRed-Express, DsRed-Express (T1), mOrange, mOrange2, mBanana, ZsYellow1, ZsGreen, ZsGreen1, Ac-GFP1, Dendra2 (switchable), Timer (switchable), AmCyan1, EYFP, EGFP, ECFP, mECFP, mCFP, GFP, EBFP, EBFP2, Sapphire, T-Sapphire, Cerulean, mcerulean, CyPet, Midori-Ishi Cyan, mTFP1 (Teal), Ac-GFP, TurboGFP, Emerald, mEmerald, Azami Green, Topaz, Venus, mVenus, mCitrine, YPet, PhiYFP, Kusabira Orange, Kusabira Orange2, mOrange, mTangerine, mRFP1, JRed, AQ143, SuperfolderGFP, sfGFP, mWasabi, TagGFP, Azurite, mTagBFP, TagCFP, TagYFP, TagRFP, TagRFP-T, mRuby, mApple, dKeima-Tandem, and mKO; optical highlighter fluorescent proteins, such as PA-GFP (G), PA-GFP (N), PA-GFP (P), PS-CFP (C), PS-CFP (G), PS-CFP2 (N), PS-CFP2 (P), PA-mRFP1 (R), PA-mRFP1 (P), CoralHue Kaede (G), CoralHue Kaede (R), wtKikGR (G), wtKikGR (R), mKikGR (G), mKikGR (R), tdEos (N), tdEos (P), dEosFP-Tandem (G), dEosFP-Tandem (R), mE-os2FP (G), mEos2FP (R), Dendra2 (G), Dendra2 (R), Dendra2 (N), Dendra2 (P), CoralHue Dronpa (G), Dronpa (P), Kindling (KFP1), and KFP1P.

However, there are embodiments where it is more useful to apply non-fluorescence markers, so that at least one of said first, second or third markers are chromogenic, colour, luminescence or infrared (see e.g. Shu et al., Science 324 (2009), 804-807) markers, preferably selected from LacZ, GUS and luciferase.

Preferably, the first, second, third and fourth polynucleotide are inserted ("knocked in") at the same site in the genome in the genotypes used according to the present invention. This is preferably a well-known and well-described and an easily accessible locus. Such a locus is often referred to as a locus that is widely used for achieving generalized expression in a given cell (or animal; e.g. in the mouse) or as a "convenient gene targeting locus". Examples for such loci are the Rosa26 locus (Soriano et al., Nat. Genet. 21 (1999), 70-71; Irion et al, Nat. Biotechnol. 25 (2007), 1477-1482), the HPRT (hypoxanthineguanine phosphoribosyltransferase; Thomas et al., Cell 51 (1987), 503-512) locus, the Col1A1 (Collagen, type I, alpha 1; McCreath et al., Nature 405 (2000), 1066-1069) locus or the beta-actin locus (Jägle et al., Genesis 45 (2007), 659-666; heterologous targeting possible). The examples according to the present invention work with the Rosa26 locus, because this is a well established strategy in mouse ES cells. Accordingly, it is preferred that the first, second, third or fourth nucleic acid molecule is integrated at a predetermined locus in the genome of the cell, preferably at the Rosa26 locus. A preferred embodiment according to the present invention is therefore characterised in that said first, second, third or fourth nucleic acid molecule is integrated at a predetermined locus in the genome of the cell, preferably at the Rosa26 locus, at the HPRT locus, at an artificially introduced locus of a yeast artificial chromosome (YAC) or a bacterial artificial chromosome, especially at the Rosa26 locus.

A preferred promoter for the marker genes is the CMV/beta-actin enhancer (CA) promoter (pCA). However, besides heterologous promoters, such as pCA or PGK, also the promoters present at the integration site can be used (e.g. the Rosa26 promoter).

A preferred embodiment of the present invention is characterised in that the (ES) cells obtained as the ninth genotype are injected into blastocysts. These blastocysts which have been manipulated by this injection are implanted into an animal suitable for giving birth to embryos derived from such ES-cell injected blastocysts to obtain animals having said ninth genotype and optionally breeding said animal having said ninth genotype with a wild type animal to further achieve homozygosity of the integrated gene of interest by breeding. Homozygosity can also be achieved by (re)targeting of already targeted ES cells with the goal to target the second (non targeted) allele.

Another aspect of the present invention is a double marker cell line, especially an embryonic stem cell line, obtainable according to the method as described above and disclosed herein.

>

Preferably, this double marker cell line has a genotype which is heterozygous wherein on one allele a promoter is operably linked to a polynucleotide sequence encoding a first marker protein and on the other allele a promoter is operably linked to a polynucleotide sequence encoding a second marker.

The minimum requirements for such a double marker cell line are two different marker genes, located on the same genetic position but each marker gene on the corresponding allelic location. Each marker comprises a promoter and a polyA tail.

Preferably, these promoters are identical and are the pCA promoter, the PGK promoter or the Rosa26 promoter.

A preferred double marker cell line according to the present invention is characterised in that it comprises a heterologous gene for a selection marker, preferably neomycin or Hygromycin, under the control of an efficient constitutive promoter, which is functioning well in the cells used in the experiment e.g. the PGK promoter.

Since the Rosa26 locus is so well characterised and described, it is preferred that said polynucleotide sequence encoding a first marker protein and said polynucleotide sequence encoding a second marker are integrated at the Rosa26 locus.

Preferably, the double marker cell line according to the present invention is murine, rat, rabbit, jack rabbit, primate, preferably human, cat, dog, sheep, goat, bovine, pig, frog, fish, insect, plant, especially moss, tobacco, maize, Arabidopsis, rice or barley, equine or guinea pig, especially murine or rat. Even more preferred, the double marker cell line according to the present invention is an embryonic stem cell line; however, there are other embodiments where provision of the cells and cell lines according to the present invention are provided as somatic cells (i.e. diploid cells which are not germline cells (gametes). Somatic cells represent all body cells apart from germ cells, gametocytes and undifferentiated stem cells. Also it is possible that somatic cells or cell lines can be reprogrammed into stem cells, so called iPS or piPS cells (induced pluripotent stem cells or protein-induce pluripotent stem cells) using the appropriate transcription factors in the respective culture media (Takahashi et. al., 2006, Cell; Wernig et. al., 2007, Nature, Zhou et al., 2009, Cell Stem Cell 4 (5), 381-384). A prerequisite to perform gene targeting in cells is to be able to culture the cells and to introduce foreign DNA into the cells. Having fulfilled these requirements, any cell can potentially be manipulated according to the present invention.

A preferred embodiment of the present invention is a double marker cell line wherein at least one of said two markers, preferably both markers, are fluorescence markers, preferably tandem dimer tomato (tdTomato), green fluorescent protein (GFP), enhanced green fluorescent protein (EGFP), red fluorescent protein (RFP), enhanced blue fluorescent protein (EBFP), enhanced cyan fluorescent protein (ECFP), yellow fluorescent protein (YFP), or orange fluorescent protein, especially said two markers are tdTomato and EGFP.

According to a preferred embodiment of the present invention, the genotypes according to the present invention can be provided with markers in two (instead of one) different predetermined loci so that four different marker genes can be used. In each of the two loci, the constructs according to the present invention can be applied with (in summary) four different marker genes. The results of the crossing events can then be visualised by investigating the presence of any one of the four different markers. In principle, this system may even be extended to three, four, five, etc., different loci, thereby allowing six, eight, ten, etc. different marker genes to be used in the system according to the present invention.

One important aim of the present invention is to provide genotypes which are obtainable with the methods as described herein which are capable of expressing a gene of interest.

The present invention is further described by the following examples and the figures, yet without being limited thereto.
Fig.1 shows the Southern Blot Strategy for the 5' external probe and EcoRI digest of genomic DNA;
Fig.2 shows confirmation of the correct targeting of the Rosa26 locus on the 5' site by Southern Blot analysis;
Fig. 3A 1) to 3) shows that fluorescence microscopy revealed that blastocysts generated after mating the original Jackson homozygous mT/mG mice to heterozygous +/mG mice (generated in house) express the expected phenotype which is 50% expressing mEGFP (A2) 100% expressing mTomato (A3); for ES cell derivation, only mTomato plus mEGFP expressing blastocysts where considered; blastocysts lacking the green fluorescence gene were discarded; in the panel 3B 1) to 3), the accomplished ES cell line is shown, expressing both, the mTomato as well as the mEGFP gene before gene targeting;

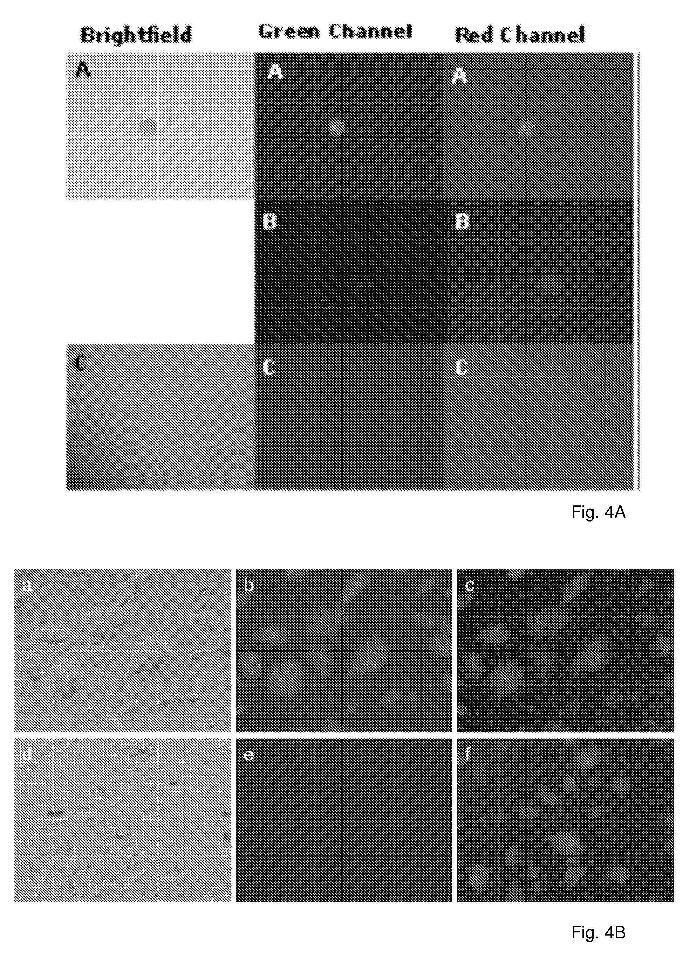

Fig.4A shows embryonic Stem Cell clones after electroporation and picking: panel (A) shows a non-targeted clone where both fluorescent loci are still present; panel (B) displays a single targeted clone lacking the EGFP expression and panel (C) the corresponding cells as expanded culture; the three pictures of Fig. 4B show a targeted clone in high magnification (40x) having lost the GFP expression signal. The picture on the left side was taken in brightfield light; ES cells and feeder cells are visible; the middle picture shows the same cells under the green fluorescent channel light with the respective GFP filter; no signal could be detected; picture on the right shows the same cells taken with red fluorescent channel light using the respective Red Fluorescence Protein filter; all ES cells express mTomato;
Fig. 5 (top) is a schematic illustration of the heterozygous genotype at the ROSA26 locus of the novel ES cell line; Fig. 5 (lower half) shows the ROSA26 targeting construct carrying a loxP flanked HygR resistance gene and two homologous arms for recombination into the ROSA26 locus indicated as red lines; the targeting construct can either recombine with the mT/mG, or the +/mG allele and therefore knock out one fluorescent locus which can be visualized as loss of one fluorescent colour in cell culture;
Fig. 6 shows that the generated male chimeric animal on the right side in each picture expresses both fluorescence genes, whereas his wildtype littermate expresses no visible fluorescence;
Fig. 7 shows the generation of the seventh and the ninth cell line according to the method of the present invention;
Fig. 8 shows the scheme of the Rosa26 targeting construct and the 3' genotyping primer locations illustrating the area to be amplified spanning the 3' targeting arm;
Fig. 9 shows the PCR analysis picture with a DNA size marker in the left side lane and DNA samples to be tested from lane 2-8 show the correct amplicon size for the clones D7, C11 and E6.

Examples:

### Materials and Methods - Holly ES Cell line

### Animal experimentation, mouse husbandry and breeding strategies

All animal experiments were discussed and approved by the institutional ethics committee. Mice were bred and maintained in makrolon cages with wood bedding (Lignocel FS 14, JRS, Germany) under SPF (Specific Pathogen Free) conditions. Animals were fed with standard rodent diet (V1126, Ssniff, Germany) and ad libitum tap water. Room temperature was 20 + 1°C and relative humidity was 50 + 10 %. The room was illuminated with artificial light from 6 am to 18 pm.

### Superovulation and mating for blastocyst production

To obtain fresh 8-cell stage embryos, 3 - 8 weeks old females (Jackson Laboratories, Bar Harbor) were induced to superovulate by intraperitoneal (i.p.) injections of pregnant mare's serum gonadotropin (PMSG; 7,5IU, Folligon^{®}, Intervet) and, 48h later, human chorionic gonadotropin (hCG; 7,5IU; Chorulon^{®}, Intervet). After hCG administration, females were exposed to appropriate males. After five days post PMSG injection, the females were sacrificed by cervical dislocation and 8-cell stage embryos were collected by flushing the oviducts of the pregnant females in M2 medium.

Mouse Lines used for breeding and subsequent ES cell line derivation

### Fluorescent Cre reporter strain

Six breeding pairs of the fluorescent Cre reporter strain Gt(ROSA)26Sor^{tm4(ACTB-tdTomato,-EGFP)Luo}/J strain (Muzumdar et al., Genesis 45 (2007), 593-605) were purchased from the Jackson Laboratories, Bar Harbour, USA (Stock Number: 007576). These homozygous mice possess a membrane-targeted tdTomato (mT) cassette flanked by loxP sites and followed by a polyadenylation signal. They express strong red fluorescence in all tissues and cell types examined. Immediately distal to the second loxP site there is an enhanced green fluorescence protein (EGFP) sequence itself followed by a polyadenylation signal. An frt-flanked neo cassette is located distal this expression vector. When bred to Cre recombinase expressing mice, the resulting offspring have the mT cassette deleted allowing expression of the membrane-targeted EGFP (mG) cassette located just downstream.

The mT/mG (membrane-Tomato/membrane-Green) targeting vector was designed with a CMV enhancer/chicken beta-actin core promoter (pCA) driving expression of the loxP flanked tdTomato sequence and the SV40 T antigen polyA signal that allows for ubiquitous and high level expression. The construct was inserted into the Gt(ROSA)26Sor locus via electroporation of (129X1/SvJ x 129S1/Sv) F1-derived R1 embryonic stem (ES) cells. Correctly targeted ES cells were injected into C57BL/6J blastocysts. Chimeric offspring was bred to outbred CD-1 mice. The resulting mutant mice were interbred to achieve homozygosity and bred to 129X1/SvJ for at least one generation to establish the colony at the Jackson Laboratories.

### Cre recombinase expressing strain

As a Cre recombinase expressing line the B6.C-Tg(CMV-cre) 1Cgn/J (Jackson Lab Stock Number: 006054) was used. This transgenic line is a deleter of loxP flanked genes in all tissues, including germ cells. The Cre gene is under the transcriptional control of human cytomegalovirus minimal promoter and is likely to be expressed before implantation during early embryogenesis. Since the Cre gene is X-linked, transgene transmission through males is restricted to female offspring.

### Breeding Strategy for Gt(ROSA)26Sor^{tm4(ACTB-tdTomato,-EGFP)Luo}/J - B6.C-Tg(CMV-cre)1Cgn/J

The aim of the breeding strategy was to establish two distinct single fluorescent strains out of the mT/mG strain purchased at the Jackson Laboratories. The first step was to breed the mT/mG animals to cre mice (B6.C-Tg(CMV-cre)1Cgn/J) to achieve recombination of the loxP flanked tdTomato gene. From 3 offspring animals 2 showed an only green fluorescent phenotype. One animal expressed green as well as the red fluorescence gene. This mosaic animal was kept locally separated from the actual green animals to avoid a mixing of lines. The mosaic line was further bred for analysis of transmission.

The mG line was expanded selecting Cre negative animals for breeding. The presence of the Cre transgene was tested by PCR using specific primers internal to the Cre coding region. It was not possible to breed the mG line to homozygosity due to perinatal lethality of homozygous offspring. All heterozygous animals were viable and fertile and used for further breeding. Only fluorescence expressing animals were selected and kept for a maintenance breeding colony.

The original mT/mG line was bred to homozygosity and kept as a maintenance breeding colony. Homozygosity was tested by a PCR protocol provided by the Jackson Laboratories. Two wildtype primers (forward + reverse) and one mutant (reverse) primer were used to indicate the presence of wildtype and/or mutant amplicons.

### Combining two different fluorescent lines - mT x mG

To generate the desired double fluorescent ES cell line, homozygous original mT/mG mice were bred to heterozygous mG mice and isolated 8-cell stage embryos from the oviducts of the respective female mice. Only red and green fluorescence expressing blastocysts were taken for further ES cell line derivation.

### ES cell line derivation and culture

Derivation of ES cell lines from blastocysts (Ying et al., Nature 453 (2008), 519-523).

8-cell stage embryos were transferred into M16 medium plus 2i (MEK inhibitor PD0325901, 1µM; GSK3 inhibitor CHIR99021, 3µM) and 100U/ml Esgro-Lif (Chemicon ESG1107) medium and incubated overnight (o/n) at 37°C at 5% CO2. This medium was exchanged next day. After two days, fluorescence emission was checked and blastocysts were transferred individually to 24-well plates (Sarstedt, Germany) and incubated o/n. All plates were treated with 0.2% Gelatine and seeded with mitomycin C-treated (Sigma) mouse embryonic DR4 fibroblasts (Applied Stem Cell), which are 4-drug resistant to Neomycin, Hygromycin, Puromycin and 6-thioguanine, before transferring blastocysts. After 4 days of daily medium change, double fluorescent blastocysts, having attached to the bottom of the dish and grown out, have been identified. Those embryos were picked, trypsinised and seeded on freshly prepared 24-well plates. 2i medium is changed in a daily routine and cells are checked for keeping pluripotency and growth. Differentiating and not growing cells were discarded. Having reached ES cell confluency, cells were trypsinised and seeded onto 6-well plates. 2i medium is changed in a daily routine. After some days of culture, 9 double fluorescent cell lines were established, frozen and in liquid nitrogen.

All further culturing of ES cells was done in ES cell medium. ES cell medium 50ml: 40ml DMEM medium (glucose 4,5g/l), without L-Glutamine (catalog no. PAA E15-009) the following catalog numbers beginning with PAA are obtainable from this company, 15% (7,5ml) fetal bovine serum, FBS (PAN Biotech, Germany), 2,5ml ES-MIX-Stock (described below), 50µl (1000 U/ml) LIF (Esgro-LIF Chemicon ESG1107, Millipore Billerica, MA, USA).
ES-MIX-Stock (250ml): 50ml Penicillin/Streptomycin 100x (PAA Laboratories GmbH, Pasching, Austria, catalog no. PAA P11-010), 50ml non-esssential amino acids (NEAA) 100x (PAA M11-003), 50ml Na-Pyruvat 100mM (PAA S11-003), 50ml β-mercaptoethanol 35µl of 14,3M (Sigma M7522) in 50ml D-PBS (PAA H15-002) and 50ml stable Glutamin 200mM (PAA M11-006).

ES cell media and culture conditions are well established in the art (see e.g., http://stemcells.nih.gov/research/nihresearch/scunit/culture.asp' #hesc), such media being available e.g. from Applied Stemcell, Menlo Park, CA, USA; Invitrogen, Millipore, Stemcell Technologies SARL, Grenoble, France; and the like companies) and their composition may be changed depending e.g. upon cell species. For instance, for culturing human ES cells a medium may be used containing essentially DMEM/F12 (Invitrogen 11330-032), 400ml, knockout serum replacer (Invitrogen 10828-028), 100ml, non-essential amino acids (Invitrogen 11140-050), 5ml, L-Glutamine (Invitrogen 25030-081), 2.5ml, and β-mercaptoethanol (Sigma 7522) 3.5µl for ca. 508 ml medium, optionally supplanted by 4ng/ml bFGF (R&D Systems Cat #233-FB), antibiotics like the above mentioned penicillin/streptomycin or the like (e.g. selection drugs like hygromycin).

### Sex determination of ES cell lines

Nine double fluorescent ES cell lines were thawed and expanded. 0.5 x 10⁶ cells of each cell line were cultured on gelatinized 6-well plates without feeder cells for several days in ES cell medium, before cells were harvested for sex determination by PCR. PCR amplification was performed on SMC (structural maintenance of chromosome) genes (Agulnik et al., Mamm. Genome 8 (1997), 134-138). The Smcx and Smcy genes were amplified from genomic DNA. Three lines were identified to be male and therefore suitable cell lines for gene targeting. Two of them were used for further subcloning.

### ES cell quality testing

To ensure pluripotency, chromosomal integrity and a mycoplasm free status, four tests were performed on the newly derived ES cells.

Alkaline Phosphatase Staining was performed using the APase Detection Kit (Chemicon). Undifferentiated cells express alkaline phosphatase when cultured under optimal conditions. Additionally, cells were stained with two different antibodies (SSEA-1, MC480; Abcam ab16285 and Oct-3/4, C-10; Santa Cruz sc-5279) to confirm the undifferentiated state of the cell lines. As secondary antibodies a Rabbit IgG and Mouse IgG+IgM HRP polymer (Abcam ab2891) and a goat polyclonal antibody (Millipore) were used. To test chromosomal integrity, the KaryoMax^{™} Kit (Invitrogen, 15212-046) was used and chromosome spreads were investigated upon the appropriate chromosome number. Moreover, all cell lines were tested negative for mycoplasm infection using VenorGeM Microplasma Detection Kit (Invitrogen).

### ES cell targeting and clone identification

### Targeting of the Red/Green ES cell line

The rapidly growing ES cells (cells that have been passaged two days before) were fed with ES cell Medium three hours before the electroporation. Just before trypsinising the cells, 10 x 60mm plates with Mouse Embryonic Fibroblasts (EMFIs), resistant to hygromycin, were prepared by washing two times with PBS and feeding with 3ml ES cell Medium per plate. The cells were trypsinised for 15 min. using 0.25% Trypsin per 10mm plate and then resuspended using a P1000 Pipetman. After resuspension 3,5ml of ES-Medium were added to each plate to inactivate the Trypsin. All cells were pooled in a 50ml Falcon tube and further resuspended by pipetting up and down 20 times. It is essential to achieve a single-cell suspension. An aliquot of 10µl of the cell suspension is used to count the cells. 10µl cell suspension plus 20µl Trypan Blue are mixed in an Eppendorf tube and pipetted onto a counting chamber. Stained cells indicate dead cells. Unstained ones are alive and relevant for counting. The original cell suspension was spinned down at 1300rpm for 3 min. The supernatant was discarded and the pellet was resuspended in fresh medium to obtain a concentration of 10⁷ cells/ml. 1ml of this cell suspension was transferred to an Eppendorf tube and spinned down at 1000rpm for 5min. The supernatant was discarded and the pellet was resuspended in 0,9ml PBS. 30µg of the prepared linearised targeting construct (eluted in embryo tested water) was pipetted into a pre-chilled 0,4cm electroporation cuvette following the transfer of the cell suspension. The targeting construct and the cell suspension were gently mixed using a P1000 pipetman. Electoporation parameters were 500uF and 220 Volt (BioRad Electroporator). The time constant was around 9,5ms. Electroporated cells were transferred to a 15ml tube containing 9ml ES Medium. Since electroporated cells are very fragile, they have to be resuspended very gently using a P1000 pipetman before transferring 1ml to each of the 10 prepared EMFI plates.

### Selection culture

After o/n incubation the cells were washed 2x in PBS and fed with ES cell Medium containing 200µg/ml Hygromycin. The optimal concentration of Hygromycin was determined by kill curve analyses on the respective lines. The selection medium was changed every day until single colonies appeared and where ready to be picked.

### Picking of ES cell clones

ES cell clones that survived the Hygromycin selection procedure and appeared as round bottomed colonies in 96-well plates, were washed with PBS and picked individually with a pipette onto a 96-well plate prepared with trypsin. Always twelve clones at once were incubated for 10 minutes at 37°C. Trypsinisation was stopped with 50µl of ES cell medium in each well. After extensive resuspension, all clones were transferred onto a 96-well plate prepared with feeder cells and ES medium for further expansion.

### Fluorescence detection

ES cell clones seeded on 96-well plates were screened for the loss of one fluorescence protein (mT or mG) expressed using a Zeiss Axiovert 200 inverted microscope (Zeiss, Germany) with a ProgRes SFSCAN Fluorescence camera and software (Jenoptik, Germany). The fluorescence filter used for the detection of mTomato was (Zeiss DsRed Filter: Ex558/Em583) and for mEGFP (Zeiss eGFP Filter: Ex488/Em509).

### Expansion of positive ES cell clones

Identified targeted clones having lost one fluorescent locus were trypsinised and transferred onto larger plates prepared with gelatine and feeder cells for expansion in ES cell medium supplemented with Hygromycin for several days. The medium was changed daily.

### DNA isolation from ES cells

Cells were harvested and washed with PBS. They were either frozen at -20°C for subsequent DNA isolation or directly resuspended in 1,2ml Proteinase K Buffer (50mM Tris/HCl pH 8, 100mM NaCl, 100mM EDTA pH 8, 1% SDS) plus 7.2µl Proteinase K (Fermentas) per 100mg cells. The solution was incubated o/n at 60°C under shaking conditions. After o/n lysis, 1 volume Phenol was added and the tube was rotated for 10 minutes and centrifuged at maximum speed for 10 minutes. The supernatant was transferred into a new tube where Phenol purification step was repeated. After this additional purification step, 1 volume of CIA (Chloroform:Isoamyl alcohol 24:1, Fluka) was added. Another 10 minutes of rotation and 10 minutes of centrifugation was followed by transferring the supernatant to new tube. To precipitate the DNA, 1 volume of 2-propanol was added. The tube was carefully inverted until the DNA became visible. With a yellow (200 µl) tip the precipitated DNA was transferred into a new tube and washed in 1ml of 70% Ethanol for at least 1h. The precipitated DNA was centrifuged at max. speed for 5 minutes and again 1ml of 70% Ethanol was added for incubation o/n at room temperature (RT). Next day the DNA was centrifuged again at max. speed for 10 minutes. The EtOH was removed as good as possible but without drying the DNA pellet. In TE buffer the DNA was dissolved o/n at RT (room temperature). The DNA is now ready to be measured using photometer and do any molecular biological analysis. Also the quality of the DNA was checked by loading it on a 0.7% Agarose gel.

### Materials and Methods for Molecular biological procedures

### PCR protocols for Cre Genotyping

The presence of the Cre transgene was tested by PCR using specific primers internal to the Cre coding region (forward 5'-CCTGGAAAATGCTTCTGTCCG-3'; (SEQ.ID.NO.1) reverse 5'-CAGGGTGTTATAAGCAATCCC-3'), (SEQ.ID.NO.2) using an annealing temperature of 65°C.

### PCR protocol for mT/mG Genotyping

Homozygosity was tested by a PCR protocol provided by the Jackson Laboratories. Two wildtype primers (forward 5'-CTCTGCTGCCTCCTGGCTTCT-3'; (SEQ.ID.NO.3) reverse 5'-CGAGGCGGATCACAAGCAATA-3') (SEQ.ID.NO.4) and one mutant (reverse 5'-TCAATGGGCGGGGGTCGTT-3') (SEQ.ID.NO.5) primer were used to indicate the presence of wildtype and/or mutant alleles. The optimal annealing temperature was 61°C.

### DNA Construct Preparation

All plasmids, transgenic constructs and knock out constructs were prepared using the protocols described below. Restriction digests are described specifically for each construct. Plasmids were supplied either on Whatman paper or sealed in plastic. Plasmids were retrieved by elution in molecular grade DNAse free water and transformation into E. coli cells for amplification.

### Cloning of the Rosa26 Targeting Construct

Components needed for the construction of the Rosa26 targeting vector; pBigT; loxP flanked PGK-neo/tpA cassette; Rosa26 genomic sequence were used as described in (Soriano et al., Nat. Gen. 21 (1999), 70-71; Srinivas et al., BMC Dev. Biol. 1 (2001), 4; 1471-213X/1/4).

The pBigT inducible cassette was modified by replacing the PGK-neo/tpA cassette with a PGK-hph (Hygromycin B phosphotransferase resistance) cassette before further cloning. The alteration was necessary to allow antibiotic selection in the Holly-ES cell line, which already contains a Neo resistance gene.

A pMSCVhyg vector, expressing a transcript that contains a hygromycin resistance gene, was used as a source of a hygromycin resistance cassette. A phosphoglycerate kinase (PKG) promoter (PPKG) controls expression of the hygromycin resistance gene (Hygr) for antibiotic selection in eukaryotic cells. pMSCVhyg also contains the pUC origin of replication and E.coli Ampr gene for propagation and antibiotic selection in bacteria. Excision of the Hygr was performed with a partial digest using EcoRI followed by a complete digest with HindIII. The partial digest was performed to excise the 1859bp fragment between the EcoRI and the HindIII sites. Since there are two EcoRI sites the partial digest enables to extract a fragment that was partially digested meaning only cut at one EcoRI site and not at the other.

### Partial Digest of pMSCVhyg with EcoRI

The resulting fragment containing the Hygr was 1859bp long and subcloned into the inducible cassette of the pBigT vector which had previously been digested also with EcoRI and HindIII to excise the PGK-neo/tpA cassette.

To make the Rosa26 genomic sequence compatible with the pBigT plasmid, pRosa26-1 was digested with XbaI, Klenow filled and a linker (PacI, SwaI, AscI) inserted. This plasmid was called pRosa26PA (Srinivas et al., BMC Dev. Biol. 1 (2001), 4). pBigT is a vector to target the ubiquitously expressed Rosa26 locus with a cassette that allows the inducible expression of any gene from the Rosa26 locus. It is meant to be used with the plasmid Rosa26PA, which contains the genomic sequence with which to target the Rosa26 locus. The multiple cloning site (MCS) of pBigT allows the insertion of any gene of interest. Further excision of the entire inducible cassette with the rare cutters PacI and AscI is possible. The previously modified pBigT vector was digested with PacI and AscI to release the inducible cassette and subsequently cloned into the pRosa26PA vector at the engineered PacI and AscI sites. The resulting pRosa26PA vector containing the inducible cassette was linearized with KpnI and used for electroporation and oocyte injections.

### Preparation of DNA constructs for electroporation

All constructs prepared for electroporation in a gene targeting experiment were enzymatically linearised and subsequently precipitated in 0.2 volumes 100% ethanol. The salt concentration provided in the restriction enzyme buffer is sufficient for precipitation. After centrifugation at 12000 g for 15 minutes the pellet was washed in 70% Ethanol and centrifuged again. The air-dried DNA pellet was eluted in embryo tested water. To ensure the integrity of the DNA, the size, concentration and quality was checked on gel and the exact DNA concentration was photometrically measured.

### Separation of fragments on agarose gel

Enzymatically treated plasmids were separated on a 1% agarose (LE Agarose, Biozym) gels prepared with TAE Buffer (50x TAE, 1L: 242 g Tris base, 57.1 ml glacial acetic acid, 37.2 g Na₂EDTA x 2H₂O, H₂O to 1 L) instead of standard buffer TBE (10x TBE, 1L: 108 g Tris base (890mM), 55 g boric acid (890mM), 40 ml 0.5M EDTA, pH 8.0, H₂O to 1L) for agarose gels. In this case TAE is preferred to TBE due to interfering effects of boric acid to the subsequent ligation reaction.

### Gel Purification of DNA fragments

The appropriate fragments are excised from the agarose gel with a scalpel. The DNA in gel slices is purified using QIAquick Gel Extraction Kit 250 (Quiagen) according to the protocol from the user manual. The concentrations of the purified fragments were measured on a 1% agarose gel before the ligation reaction can be started.

### DNA sample preparation from ES cells

ES cells were harvested by trypsinising the cells with 0.25% Trypsin/EDTA (Invitrogen) for 10'. Trypsin was inactivated using EMFI Medium. Cells were resuspended and plated again on a feeder free cell culture dish for 30'. This was done to let the feeder cells attach to the dish and harvest ES cells, which do not attach within this time frame, to a higher percentage. After centrifugation of the cells at 1300 rpm for 3', resuspension of the pellet with PBS and another quick spinning step the cell pellet was froze at -20°C and stored for further DNA isolation.

### DNA sample preparation from tissue

All animal tissues used, were either stored at -20°C or immediately used for DNA isolation.

### Phenol/Chloroform DNA isolation of genomic DNA

Cells or tissue were incubated in 1,2ml Proteinase K Buffer (50 mM Tris/HCl pH8, 100 mM NaCl, 100 mM EDTA pH 8, 1 % SDS) and 7,2µl Proteinase K (20mg/ml) per 100mg cells or tissue at 60°C in a shaker overnight (o/n). Next day 1 volume Phenol/Chloroform (25 vol phenol, 24 vol chloroform) was added and the tubes were rotated for 10'. After a 10' centrifugation step at maximum speed the upper phase was transferred into new tubes. This Phenol/Chloroform step was repeated once to ensure high purity of the DNA. The supernatant was mixed with CIA (24 vol Chloroform, 1 vol Isopropanol) and rotated for 10'. After centrifugation at maximum speed and RT the supernatant was transferred to a new tube. One volume of isopropanol was added. By inverting the tubes carefully the precipitated DNA becomes visible. The DNA was centrifuged at maximum speed for 10'. The supernatant was taken off and the pellets were washed in 70% Ethanol for at least 1 hour at RT. To further wash the pellets, Ethanol was replaced by fresh 70% Ethanol and the DNA was incubated o/n at RT. Next day all ethanol (EtOH) was taken off. Pellets were incubated with open lid at RT for 5 - 10'. It is important to not completely dry the pellet because it will not be solvent anymore if overdried. The pellets were eluted in TE (20µl - 200µl depending on the size of the pellet). After o/n incubation at RT the samples were photometrically measured to estimate DNA concentrations.

### Southern Blot Analysis

Genomic DNA (10µg), derived from ES cell clones, was digested over night with 3 units per µg of DNA at 37°C in a volume of 40µl. The digested samples were loaded on a 0.7% agarose gels in Tris (890mM), borate (890mM), EDTA (0,5M), pH 8.0 buffer and run at 50V overnight. The gels were incubated for 12-20 min in 0.25M HCl, washed 2x in water, denaturated for 15-20 min in 0.5M NaOH; 1.5M NaCl, washed 2x in water, neutralised for 15-20 min in 0.5M Tris/HCl pH 7.5, 1.5M NaCl, washed 2x in water and incubated for 20-30 min in 10xSSC and blotted for 16h with 20xSSC on Amersham HybondTMXL membranes (GE Healthcare). After blotting the membranes were baked at 80°C for 2h and stored at room temperature. For hybridization the membranes were preincubated in Church buffer (1% BSA, 1mM EDTA, 0.5M phosphate buffer, 7% SDS) for 2h at 68°C under rotation. The primer sequences for generating the Rosa26 5' external probe (550bp)were
Forward Primer 5' Probe: 5'-GAGCACAGGAACAATTGGC-3' (SEQ.ID.NO.6) Reverse Primer 5' Probe: 5'-AGACATTACCTTCGCCACC-3' (SEQ.ID.NO.7)

The probe was isolated as a PCR product from agarose gel and purified using a Quiaquick Gel Isolation Kit (Quiagen).

The PCR product used as hybridization probe was heat denatured and labeled with P³² marked α-dATP (Hartmann Analytic) using the Prime It II labeling Kit (Stratagene). Labeled probe DNA was purified on MicroSpinTM columns (GE Healthcare), heat denaturated, added to the hybridization buffer and rotated at 68°C 18-24h. With low stringency buffer (0.5% BSA, 1mM EDTA, 40mMphosphate buffer, 5% SDS) the membrane was washed twice for 10 minutes at room temperature. With the high stringency buffer (1mM EDTA, 40mM phosphate buffer, 1% SDS) the membrane was washed twice for 10 minutes at 68°C. Next, the membrane was exposed at -80°C to HyperfilmsTM (GE Healthcare) for 1-7 days until development. Autoradiographs were scanned and segments were excised with Adobe Photoshop software.

### Southern Blot Strategy

The 5' external probe according to Nyabi et al. (NAR 37 (2009), e55) was used.

Sequence for the 5' external probe:

5′AGACATTACCTTCGCCACCCCCACCTCACATTCTTGGACTCCCTGTGGCGTATGCCCCAGTA
TCCTTGCGATATCCCTGTTGAATTTGTAGGGTTCTAGCCTGGAATTGAGCCAGATTTT-

GTCGTTTTCCCTGCCTAGGGCGTGGAGAGCGATCTTTTAAGAACGACTCTAAAGAAGAGGGG-
GAGGGATATGCCCCTATGAATCCGTGCAAGCCAAGATCCTAGGTAAATGGTAGTGGCAAAA-
GCCCTGGTTTTAGTAGGTAATCGCTCAGAAGGAAAGTTTTAACTCCTGGGAAAGCCCCTTC-
TATGCTAATTTATTCTATCGGGCGCCTTTGCCTTTCTCATTCTTAGGAA-
TAAGGATCTTTCTCCTGTGTACTGATGCTG-
CAGCCCGCCCTGCTGGCCTGGGTCAGGGGTGCGTGGATGACCTG-
CATTTTCTGCTTTCTCATGTTACTTGTGTAACGTCTTCACCAG-
CAACTCATTTTTCGCCCAGAGCTCTGGGCTCTGGGTTTTGTCTATACCTGGATTCCTTTT-
GCCAATTGTTCCTGTGCTC-3′(SEQ.ID.NO.8)

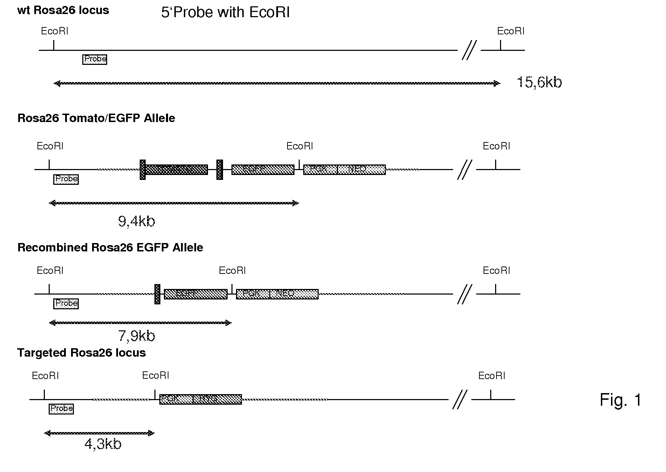

To confirm the 5' integration site, the 5' external probe (550bp) and EcoRI digests of genomic DNA was performed (15,6kb wt allele from fibroblast background, 9,4kb Tomato allele, 7,9kb EGFP allele, 4,3 kb targeted allele). The Southern Blot Strategy is also depicted in Fig. 1.

Correct targeting of the ROSA26 locus on the 5' site was confirmed by Southern Blot analysis using ES cell genomic DNA. The external probe (5' - 550bp, SEQ ID No. 8) was used to confirm the correct recombination of the homologous arm.

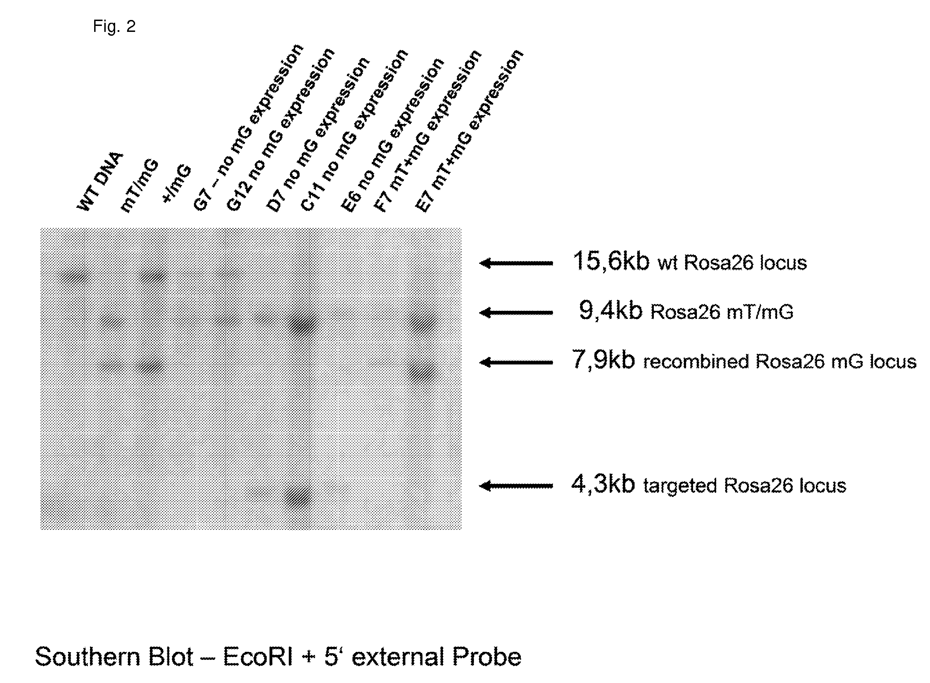

Fig. 2 shows the Southern Blot results using EcoRI as restriction enzyme and the 5' external probe to bind outside the 5' short homologous arm of the targeting construct. Wildtype samples (WT, mT/mG and +/mG were isolated from tails) served as control samples. Potentially targeted ES cell clone DNA (G7, G12, D7, C11 and E6) did not express EGFP after electroporation with the targeting construct and antibiotic positive selection. Samples F7 and E7 carry a randomly integrated targeting construct and still express both, mTomato and mEGFP genes. They were also used as control samples in this experiment. Wildtype bands in ES cell clone samples (G7, G12) origin from the feeder cell background.

Three (D7, C11, E6) out of five potentially targeted clones could be identified as correctly targeted via Southern blot analysis on the 5' end. Two clones that have lost the EGFP expression do not show the correct targeted band on the Southern Blot. However, they do not exhibit the mEGFP band either. The 5' integration site of the targeting vector must have integrated in a different way.

### PCR protocol for 3' Rosa26 Targeting

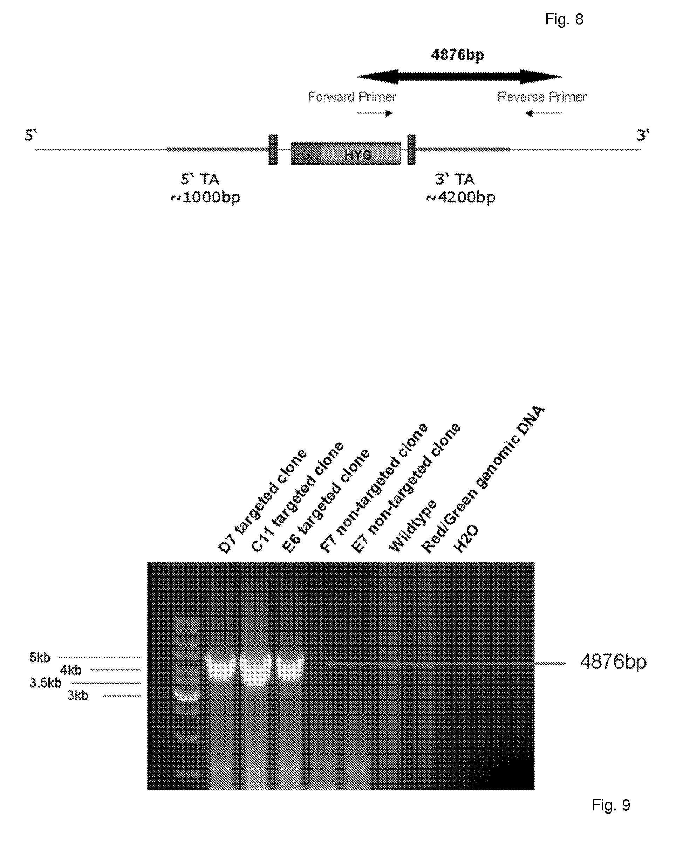

To confirm the 3' integration site after gene targeting the Holly ES cell line, a long range PCR was designed. The forward primer
5'-CCTCGACCTGCAGCCCAAGC-3' (SEQ. ID. NO. 9)
was positioned in the Hygromycin resistance cassette. The reverse primer
5'- GCAGTGTTGAGGGCAATCTGGGA-3' (SEQ. ID. NO. 10)
was placed outside the 3' Targeting arm of the Rosa26 targeting construct. The desired amplicon is 4876bp in length and proves the targeted integration of the construct on the 3' side. The scheme of the Rosa26 Targeting construct and the 3' genotyping primer locations illustrating the area to be amplified spanning

the 3' targeting arm (see Fig. 8).

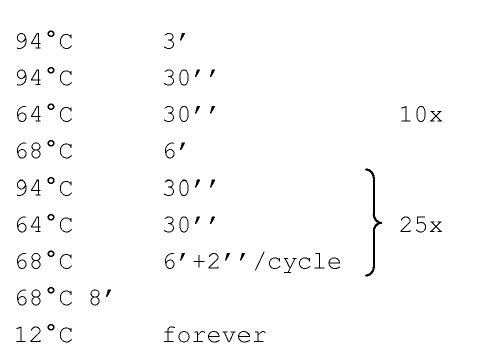

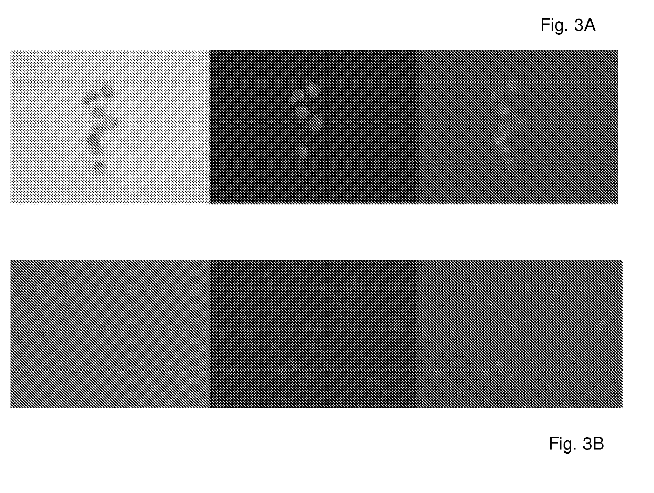

Figure 3: (A 1 - 3) Fluorescence microscopy revealed that blastocysts generated after mating the original Jackson homozygous mT/mG mice to heterozygous +/mG mice (generated in house) express the expected phenotype which is 50% expressing mEGFP (A2) 100% expressing mTomato (A3). For ES cell derivation, only mTomato plus mEGFP expressing blastocysts where considered. Blastocysts lacking the green fluorescence gene were discarded. In the panel (B1 - 3), the accomplished ES cell line is shown, expressing both, the mTomato as well as the mEGFP gene before gene targeting.

Figure 4A: Embryonic Stem Cell clones after electroporation and picking: panel (A) shows a non-targeted clone where both fluorescent loci are still present. Panel (B) displays a single targeted clone lacking the EGFP expression and panel (C) the corresponding cells as expanded culture.

Figure 4B: The three pictures of Fig. 4B show a targeted clone in high magnification (40x) having lost the GFP expression signal. The picture on the left side was taken in brightfield light; ES cells and feeder cells are visible; the middle picture shows the same cells under the green fluorescent channel light with the respective GFP filter; no signal could be detected; picture on the right shows the same cells taken with red fluorescent channel light using the respective Red Fluorescence Protein filter; all ES cells express mTomato.

### ROSA26 knock-in gene targeting experiments

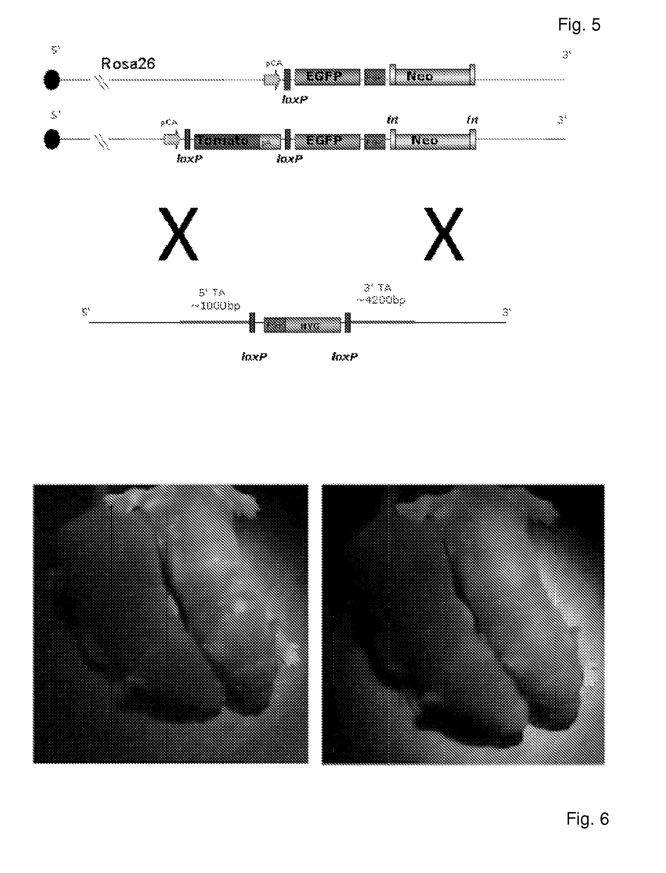

First, a ROSA26 targeting construct was designed using the two-step cloning procedure developed by (Srinivas, Watanabe et al. 2001), only carrying a Hygromycin resistance gene for positive selection (Figure 3B). The targeting scheme is outlined in Figure 5.

Two independent targeting experiments were performed. The antibiotic selection was performed during a 6-7 day culture under Hygromycin B treatment. The surviving clones were picked and separately transferred to four 96-well plates. After a 2-3 days culture, the clones were examined for fluorescence expression signals. In total, six clones having lost the mEGFP expression, suggested positive targeting events at the recombined mEGFP RO-SA26 allele. All non-targeted clones still expressed strong mTomato as well as mEGFP expression. Taken together, in four 96-well plates, six clones having lost mEGFP fluorescence expression could be identified and were chosen for further expansion. Also clones which have not lost any of the fluorescence loci were chosen as control clones and further expanded for additional analysis to confirm HR.

Figure 5: (A) Schematic illustration of the heterozygous genotype at the ROSA26 locus of the novel ES cell line and the targeting construct. (B) The ROSA26 targeting construct is carrying a loxP flanked HygR resistance gene and two homologous arms for recombination into the ROSA26 locus indicated as red lines. The targeting construct can either recombine with the mT/mG, or the +/mG allele and therefore knock out one fluorescent locus which can be visualized as loss of one fluorescent colour in cell culture.

### Southern Blot Analysis to confirm Gene Targeting

Correct targeting of the ROSA26 locus was confirmed by Southern blot analysis using ES cell genomic DNA. An external 5' probe (550bp) was used to confirm the correct recombination of the 5' homologous arm.

### Germ-line transmission of the double fluorescent ES cell line

To test whether the created ES cell line would contribute to embryonic tissue and moreover be transmitted via the germline, an ES cell injection experiment was conducted. The double fluorescent ES cell line was tested by injecting non targeted double fluorescent ES cells into blastocysts. These manipulated blastocysts were surgically transferred into pseudopregnant females for in vivo growth. Out of this one experiment, one male chimeric animal could be generated (Figure 6) proving the potency of the line to contribute to embryonic tissue. Further breeding of this chimera to two wildtype CD1 females resulted in 23 pups which were all not expressing any fluorescence. At least one offspring animal that expresses 100% red/green fluorescence would have proven germline transmission. Additional blastocyst injection experiments have to be performed to proof potential germline transmission of the double fluorescent ES cell line.

Figure 6: The generated male chimeric animal on the right side in each picture expresses both fluorescence genes, whereas his wildtype littermate expresses no visible fluorescence.

### PCR Analysis to confirm Gene Targeting on the 3' side

The 3' end of the targeted locus was confirmed by a long range PCR Analysis spanning the 3' targeting arm of the targeting construct. The clones being identified as correctly targeted on the 5' end via Southern Blot analysis (D7, C11, E6) were considered for PCR analysis and exhibit the correct amplicon size (4876bp). The control ES cell DNA samples F7 and E7, which still express red and green fluorescence, show no signal at the desired position as well as the wildtype tissue DNA sample and DNA of a Rosa26 - Red and Green fluorescence expressing animal. Fig. 9 shows the PCR analysis with a DNA size marker in the left side lane and DNA samples to be tested from lane 2-8. Clones D7, C11 and E8 show the correct amplicon size.

### Example 1:

Generation of a double fluorescent ES cell line (Holly ES cell line) out of Gt(ROSA)26Sor^{tm4(ACTB-tdTomado,-EGFP)Luo}/J strain (Muzumdar et al. 2007). The mouse strain is available commercially at the Jackson Laboratories in Bar Harbor, USA, with the Stock Number 007576 or 007676.

To improve the efficiency of Rosa26 knock-in gene targeting experiments in murine ES cells, a double fluorescent ES cell line was designed to visualize targeting events by fluorescence gene expression or lack of expression.

To obtain the cell line, the Gt(ROSA)26Sor^{tm4(ACTB-tdTomado,-EGFP)Luo}/J strain was bred to the X-linked Cre expressing strain B6.CTg(CMV-cre)lCgn/J (Jackson Laboratory Stock Number 006054) and achieved Cre recombination of the loxP flanked mTomato fluorescence gene. Hence, two different fluorescent strains, the mT/mG original line expressing mTomato and the +/mG - Cre negative strain expressing mEGFP were kept as separate stocks. Eventually, the two fluorescent lines were crossed to achieve double fluorescent early embryos for ES cell derivation (Fig.3). The ES cell line (*Holly* ES cell line), carrying the mTomato fluorescence gene on one ROSA26 allele and the mEGFP gene on the other allele, was used to conduct two targeting experiments to prove the principle of visualizing a targeting event by the loss of one of the two fluorescent alleles by knocking in a ROSA26 targeting construct (Fig. 5).

A schematic representation of the method according to the present invention and example 1 is given in Fig. 7 (the red arms represent the homologous arms for homologous recombination: Starting from the homozygous Gt(ROSA)26Sor^{tm4(ACTB-tdTomato,-EGFP)Luo}/J strain (Muzumdar et al. 2007) wherein tdTomato is the first marker and EGFP is the second marker, the neomycin gene (with the PKG promoter) acting as a selection marker (between frt sites), as a first genotype (wherein only tdTomato is expressed by control of the pCA promoter), this first genotype is crossed with the X-linked Cre expressing strain B6.CTg(CMV-cre)1Cgn/J as the second genotype. This second genotype comprises Cre recombinase controlled by the CMV promoter heterozygously on the X chromosome. Breeding of the first and the second genotype results in the third genotype wherein the EGFP (as the second marker) is under the control of the pCA promoter and wherein the Cre recombinase is present on the X chromosome (see Fig. 7A).

Then the third genotype is bred to a fourth genotype (which is in this example identical with the third genotype) to select for Cre recombinase negative genotypes. The genotype wherein the Cre recombinase is not present anymore is the fifth genotype. This fifth genotype is crossed with the sixth genotype wherein tdTomato is present as the third marker and, of course, EGFP is the second marker is present. In the present example, the sixth genotype is identical to the first genotype (see Fig. 7B).

The result of the crossing of the fifth genotype with the sixth genotype is the seventh genotype (the cell line according to claim 1), a heterozygous genotype wherein on one allele, the second marker (EGFP) is under the control of the pCA promoter and on the other allele, the third marker (tdTomato) is under the control of the pCA promoter. This genotype was taken for ES cell derivation. For introducing a gene of interest into this seventh cell line, the eighth genotype (the gene targeting DNA construct carrying the gene of interest; in the case of the present example, a Hygromycin selection marker; however, this can be any gene of interest which is not naturally occurring in this position in the genome of the host species (in the present case: mouse)) is introduced into the cells of the seventh cell line. In the case of a successful targeting event the homologous arms of the targeting construct naturally recombine with the homologous sequence in the genome of the double fluorescent ES cells (seventh genotype). The homologous sequence in the double fluorescent cells flank the marker and selection genes. The homologous arms on the targeting construct (eighth genotype) flank the gene of interest. The successful targeting event results in the ninth cell line for which two possibilities exist: in the first possibility, the second marker (EGFP) is under the control of the pCA promoter, whereas the first and third marker have been replaced by homologous recombination and the gene of interest has inserted in the same location; in the second possibility, the third marker (tdTomato; = also the first marker) is under the control of the pCA promoter (Fig 7C), whereas the second marker has been replaced by homologous recombination and the gene of interest has inserted in the same location.

The result directly obtained from the method of the present invention is a gene of interest, present in one of the two possibilities of the ninth genotype. If homozygosity of the gene of interest if preferred, the targeted ES cells (ninth genotype) can be injected into wildtype blastocysts. The respective blastocysts can be transferred into pseudopregnant foster mothers. Chimeric offspring (a mix between targeted red or green cells and wildtype cells) can be further bred to wildtype animals. The germline compatible targeted cells are inherited to offspring and animals are born as heterozygous, red or green animals. These heterozygous animals can be bred among each other (Fig. 7D and E, respectively) resulting in 25% offspring, homozygous for the gene of interest. Homozygosity can also be achieved in vitro by (re)-targeting of already targeted ES cells with the aim to target the second (non targeted) allele. In both cases, offspring or cells homozygous for the gene of interest will have lost both fluorescent loci.

### Example 2:

Generation of a double marker ES cell line by gene targeting a wildtype ES cell line

To generate a double marker ES cell line, a wildtype ES cell line (e.g. from Open Biosystems, CatNo.: MES1393)is used to target two desired marker genes into two corresponding alleles on one desired locus. The targeting experiments can be conducted at once by introducing two different targeting constructs within the same targeting experiment and select for double marker clones which have to be verified by PCR and Southern Blot analysis for correct integration.

Alternatively, two serial targeting experiments on the same ES cell line can be conducted to produce the desired double ES cell line.

The targeting constructs are constructed using the two-step cloning procedure developed by (Srinivas, Watanabe et al. 2001). The constructs are designed to target the Rosa26 locus. The targeting constructs are designed to comprise a promoter to drive marker gene expression (e.g. CMV early enhancer/chicken β actin promoter (CAG promoter)), a marker gene (e.g. Enhanced Green Fluorescent Protein (EGFP)), and a selection gene for selection of targeted ES cell clones (e.g. Hygromycin under the control of a PGK (Phosphoglycerate Kinase Gene) promoter). It is also possible to use the endogenous Rosa26 promoter to drive marker gene expression instead of a promoter being directly located on the targeting construct. In this case, the construct is targeted directly 3' to the Rosa26 promoter so that the genes coding for the marker and optionally, the selection marker are transcribed from the Rosa26 promoter.

### Targeting of ES cells

The rapidly growing ES cells (cells that have been passaged two days before) are fed with ES-Medium three hours before the electroporation. Just before trypsinising the cells, 10 x 60mm plates with Mouse Embryonic Fibroblasts (EMFIs), resistant to the antibiotic being present on the targeting construct (e.g. Hygromycin), are prepared by washing two times with PBS and feeding with 3ml ES-Medium per plate. The cells are trypsinised for 15 min. using 0.25% Trypsin per 10mm plate and then resuspended using a P1000 Pipetman. After resuspension 3,5ml of ES-Medium is added to each plate to inactivate the Trypsin. All cells are pooled in a 50ml Falcon tube and further resuspended by pipetting up and down 20 times. It is essential to achieve a single-cell suspension. An aliquot of 10µl of the cell suspension is used to count the cells. 10µl cell suspension plus 20µl Trypan Blue are mixed in an Eppendorf tube and pipetted onto a counting chamber. Stained cells indicate dead cells. Unstained ones are alive and relevant for counting. The original cell suspension is spinned down at 1300rpm for 3 min. The supernatant is discarded and the pellet resuspended in fresh medium to obtain a concentration of 10⁷ cells/ml. 1ml of this cell suspension is transferred to an Eppendorf tube and spun down at 1000rpm for 5min. The supernatant is discarded and the pellet is resuspended in 0,9ml PBS. 30µg of the prepared linearised targeting construct (eluted in embryo tested water) is pipetted into a pre-chilled 0,4cm electroporation cuvette following the transfer of the cell suspension. The targeting construct and the cell suspension are gently mixed using a P1000 pipetman. Electoporation parameters are 500uF and 220 Volt (BioRad Electroporator). The time constant is around 9,5ms. Electroporated cells are transferred to a 15ml tube containing 9ml ES Medium. Since electroporated cells are very fragile, they have to be resuspended very gently using a P1000 pipetman before transferring 1ml to each of the 10 prepared EMFI plates.

### Selection culture

After o/n incubation the cells are washed 2x in PBS and fed with ES-Medium containing the respective antibiotic. The optimal concentration of antibiotic is determined by kill curve analyses on the respective lines. The selection medium is changed every day until single colonies appear and are ready to be picked.

### Picking of ES cell clones

ES cell clones that survived the antibiotic selection procedure, express the desired marker genes and appear as round bottomed colonies in 96-well plates, are washed with PBS and picked individually with a pipette onto a 96-well plate prepared with trypsin. Always twelve clones at once are incubated for 10 minutes at 37°C. Trypsinisation is stopped with 50µl of ES medium in each well. After extensive resuspension, all clones are transferred onto a 96-well plate prepared with feeder cells and ES medium for further expansion. Marker gene expression can also be checked in the 96-well plate after picking and culturing.

The correct location of the introduced targeting constructs can be determined by Southern Blot and PCR analysis. Having identified a correctly targeted clone allows further expansion of the cell line. After creation of a ES cell line successfully targeted with a single marker, the targeting is repeated using a targeting construct comprising a second marker and a second antibiotic resistance gene (e.g. a promoter to drive marker gene expression (e.g. CMV early enhancer/chicken β actin promoter (CAG promoter)), a marker gene (e.g. Red Fluorescent Protein (RFP)), and a selection gene for selection of targeted ES cell clones (e.g. Neomycin under the control of a PGK (Phosphoglycerate Kinase Gene) promoter) so as to create the double marker cell line. When introducing both markers comprised within their respective targeting construct at the same time, the resulting targeted cell line must be carefully checked for the correct insertion of both marker genes at the Rosa26 locus, one at each allele.

The achieved double marker ES cell line can be aliquoted and frozen for subsequent double fluorescent targeting experiments, as described herein under example 4.

### Example 3:

Generation of a double marker ES cell line by using two single fluorescent mouse strains

In this third approach, single fluorescent mouse strains can be crossed, resulting in a double fluorescent strain which can be applied to ES cell derivation.

Single fluorescent mouse strains are available at the Jackson Laboratories, Bar Harbor, USA. The Stock Numbers 006331 or 006071 are both expressing EGFP, located in the Rosa26 locus. Stocks that are available carrying Tomato on the Rosa26 locus, only express Tomato upon Cre recombination and are available among the stock numbers (007914, 007909, 007905). Any marker combination that allows visual distinction (e.g. EGFP vs. Tomato) is possible.

Before crossing two distinct marker strains, a breeding step with a Cre expressing animal may be necessary to start marker expression (see stock numers 007914, 007909, 007905 for Tomato expression). Having two distinct actual marker expressing strains available, these strains can be crossed by mating, resulting in a double marker strain. It is expected that 25% of the offspring will express both markers. These animals should be heterozygous at the Rosa26 locus, carrying a different marker gene at each allele.

Blastocysts from this double marker strain (e.g. EGFP/Tomato) can be used to derive a double fluorescent ES cell line. The ES cell derivation procedure can be conducted as described in Materials & Methods, "Derivation of ES cell lines from blastocysts (Ying et al., Nature 453 (2008), 519-523)". Briefly, 8-cell stage embryos are cultured, blastocysts derived from the inner cell mass and individualized for further culturing. After selection for adherence, growth and marker expression, an ES cell line is established.

The generated double marker ES cell line (seventh cell line) can be used for knock-in gene targeting experiments, as described hereinbelow (example 4).

It is readily apparent to one of ordinary skill in the art that a combination of the above described approaches for generating a double marker cell line is possible, without deviating from the concept of the invention. For instance, a single marker cell line created as described in example 2 may be used to create an animal heterozygous or homozygous for that marker. This animal can then be crossed with a single marker animal as described in example 3 to arrive at a double marker animal which can then be further used to derive a double marker ES cell line. Moreover, a single marker animal may be used to derive an ES cell line as described hereinabove and in example 2, which can then be targeted with a second marker gene to create a double marker ES cell line. Further other variations will be apparent to the person of ordinary skill in the art.

### Example 4

This Example comprises the technical description of the gene targeting procedure that can be applied on the double fluorescent ES cell line, preferably generated by using the methods described in example 1, 2 or 3, or a combination thereof.

### Targeting double marker ES cell line

To conduct a Rosa26 knock-in gene targeting experiment, a Rosa26 targeting construct (eighth genotype) has to be designed carrying two homologous arms and an antibiotic resistance gene which is different from both antibiotic resistance genes used in the previous gene targeting experiments to generate this double marker ES cell line. The two-step cloning procedure developed by (Srinivas, Watanabe et al. 2001) can also be applied here. To study the expression of a gene of interest (GOI), which can be any gene of interest which is not naturally occurring in this position in the genome of the host species (in the present case: mouse), the according GOI can be introduced into the targeting construct under the transcriptional control of a promoter located on the targeting construct or using the endogenous Rosa26 promoter. In a successful targeting experiment, the targeting construct can either recombine with the first marker on the Rosa26 allele or with the second marker in the Rosa26 allele. In the first case, the expression of the first marker will be destroyed by knock-out and replaced by expression of the expression cassette present on the targeting construct (eighth genotype) resulting in the first possibility of the ninth cell line. In the second case, the expression of the second marker will be destroyed by knock-out and replaced by expression of the expression cassette present on the targeting construct (eighth genotype) resulting in the second possibility of the ninth cell line. It is also possible, that two gene targeting constructs recombine on both Rosa26 alleles. This would result in the loss of both marker genes in the ES cell line.

### Southern Blot Analysis to confirm Gene Targeting

Correct targeting of the ROSA26 locus can be confirmed by Southern blot analysis using ES cell genomic DNA. Recombination can also be tested by PCR analysis.

### Germ-line transmission of the double fluorescent ES cell line

To test whether the created ES cell line would contribute to embryonic tissue and moreover be transmitted via the germline, an ES cell injection experiment is conducted. The double fluorescent ES cell line is tested by injecting non targeted double fluorescent ES cells into blastocysts. These manipulated blastocysts are surgically transferred into pseudopregnant females for in vivo growth. Out of this experiment, a male chimeric animal can be generated, proving the potency of the line to contribute to embryonic tissue.

The result directly obtained from the method of the present invention is a gene of interest, present in one of the two possibilities of the ninth genotype. If homozygosity of the gene of interest if preferred, the targeted ES cells (ninth genotype) can be injected into wildtype blastocysts. The respective blastocysts can be transferred into pseudopregnant foster mothers. Chimeric offspring (a mix between targeted red or green cells and wildtype cells) can be further bred to wildtype animals. The germline compatible targeted cells are inherited to offspring and animals are born as heterozygous, red or green animals. These heterozygous animals can be bred among each other (Fig. 7D and E, respectively) resulting in 25% offspring, homozygous for the gene of interest. Homozygosity can also be achieved in vitro by (re)-targeting of already targeted ES cells with the aim to target the second (non targeted) allele. In both cases, offspring or cells homozygous for the gene of interest will have lost both fluorescent (marker) loci.

While the present invention has been fully described hererinabove, it will be apparent to those of ordinary skill in the art that numerous modifications, including, but not limited to, variations in size, materials, shape, form, function and manner of operation, assembly and use may be made, without departing from the principles and concepts of the invention as set forth in the claims.

For instance, the marker used in the practice of the invention is generally a gene encoding a marker protein, whose expression can be preferably detected by optical means. However, other proteins can be envisaged that may be detected by other than optical means. Moreover, the marker may not even necessarily be a protein, but could be an RNA or even consist in properties of the polynucleotide DNA sequence of the marker itself. For instance, a gene encoding for a functional RNA such as RNAi, siR-NA, microRNA, antisense RNA, and the like, may be used as a marker where such functional RNA itself specifically targets and modifies the expression of another gene within the same cell (line) which modification is then detected. Furthermore, by the same token, a DNA marker is a functional DNA sequence, such as an enhancer or a silencer sequence, which may act in cis or in trans, and the functional presence of which can be detected by its specific modification or the expression of another gene in the same cell (line). Such other gene may comprise any homologous or heterologous gene giving rise to an easily detectable phenomenological change, such as antibiotic resistance, a change in color or a membrane-expressed protein which may itself be detected by staining using e.g. a dye-coupled specific antibody. Many detection methods may be used and are known to those of skill in the art (see e.g., US 2003/0135872 A1, Wood, 1995, Curr. Opin. Biotechol. 6, 50-58, 1995)

The numbering given herein to genotypes, cell lines, markers proteins sequences or promoters and the like, is not necessarily meant to reflect a given order in which those elements should be used or obtained, but rather serves the identification of a particular element when referenced later and the distinction from other elements which may be mentioned in the same paragraph or for description of the same embodiment of the invention.

Preferably, the present invention is defined as in the following embodiments:
1. A method for producing a double marker cell line comprising the steps of
   - providing a fifth genotype comprising, within the background of a first genotype (preferably wild-type) a promoter operably linked to a second polynucleotide sequence encoding a second marker;
   - crossing the fifth genotype with a sixth genotype, the sixth genotype containing a third polynucleotide comprising
      (a) a promoter operably linked to
      (b) a third polynucleotide sequence encoding a third marker heterologous to the first genotype,
   - selecting offspring comprising the first polynucleotide sequence encoding for the first marker protein as well as the third polynucleotide sequence encoding for the third marker protein, thereby obtaining a seventh genotype which comprises the second polynucleotide as well as the third polynucleotide,
      and
   - obtaining a double marker cell line, especially a double marker embryonic stem (ES) cell line, from said seventh genotype.
   The invention further provides the above method wherein the flanking sequence to the 5' of said first polynucleotide and the flanking sequence to the 5' said third polynucleotide are substantially identical and substantially unique within the genomic background of the double marker cell line, and the flanking sequence to the 3' of said first polynucleotide and the flanking sequence to the 3' said third polynucleotide are substantially identical and substantially unique within the genomic background of the double marker cell line. Preferably, one of the first or third polynucleotide sequences is located on the maternal chromosome, while the other of the first and third polynucleotide sequences is located on the paternal chromosome.
   The design of the 5' and 3' flanking sequences that are homologous to the intended genomic target site is known to a person of ordinary skill in the art (see e.g. the textbook "Manipulating the Mouse Embryo" 3rd Ed. (2003), cited hereinabove).
2. A method for producing a double marker cell line comprising the following steps:
   - providing a fifth genotype, being a cell line, comprising at a defined locus a promoter operably linked to a second polynucleotide sequence encoding a second marker;
   - introducing, by gene targeting, into the fifth genotype a third polynucleotide comprising
      (a) a promoter operably linked to
      (b) a third polynucleotide sequence encoding a third marker heterologous to the first genotype,
   - thereby obtaining a seventh genotype being a cell line, which comprises the second polynucleotide as well as the third polynucleotide,
      and
   - obtaining a double marker cell line, especially a double marker embryonic stem (ES) cell line, from said seventh genotype.
   Preferably, the flanking sequence to the 5' of said first polynucleotide and the flanking sequence to the 5' said third polynucleotide are substantially identical and substantially unique within the genomic background of the double marker cell line, and the flanking sequence to the 3' of said first polynucleotide and the flanking sequence to the 3' said third polynucleotide are substantially identical and substantially unique within the genomic background of the double marker cell line.
   Preferably, one of the first or third polynucleotide sequences is located on the maternal chromosome, while the other of the first and third polynucleotide sequences is located on the paternal chromosome.
   The design of the 5' and 3' flanking sequences that are homologous to the intended genomic target site is known to a person of ordinary skill in the art (see e.g. the textbook "Manipulating the Mouse Embryo" 3rd Ed. (2003), cited hereinabove).
   The invention further provides the above method under 1 or 2 wherein the marker is a DNA marker, an RNA marker, or a protein marker. The RNA marker may be any RNA sequence which upon expression within a cell is able to produce a detectable phenotype. This may be caused by an antisense RNA, an RNAi, an ncRNA, an RNAzyme, a Ribozyme, or the like RNA molecule able to effect a protein, RNA, or gene within that cell so as to produce a detectable phenotype. The DNA marker may be any DNA sequence which upon expression within a cell is able to produce a detectable phenotype. This may be caused by a DNA sequence acting in cis, that is, on neighbouring DNA elements, or in trans, that is, on DNA elements located farther away or even on another chromosome. Such DNA sequence may comprise elements such as an enhancer, a silencer or other suitable sequence known to one of skill in the art able to modify a gene or its activity. The modified gene or gene activity in turn leads to a phenomenological change which can be detected.
   It is understood by one of skill in the art that the protein, RNA, or gene within the cell that is influenced by the marker of the invention and whose altered state may be phenomenologically detected, may be homologous to that cell, i.e. exists in the wild type cell (line) or animal, or heterologous, in which case it has been introduced by crossing, breeding or genetic modification, such as knock-in, transfection or the like technique able to establish a stable genetic change within a cell line or animal.
   The invention also provides the method where the marker protein is a fluorescent marker protein as exemplified described hereinabove.
   The invention further provides the above method wherein the first and third polynucleotide sequences are located at the same locus within the genome, so that the double marker cell line is heterozygous with respect to that locus, comprising the third polynucleotides sequence at one allele and the first polynucleotide sequence at the other allele.
   The invention also provides the above method where the double marker cell line is established from an embryonic cell derived from a blastocyst of said seventh genotype where said seventh genotype is an animal. The invention also provides the above method wherein the double marker cell line is derived from a somatic cell of said seventh genotype. The invention further provides the above method where the said somatic cell is further induced, to become an induced pluripotent stem cell.
3. A method for producing a double marker cell line comprising the following steps:
   - providing a first genotype containing a first polynucleotide comprising
      (a) a promoter operably linked to
      (b) a first polynucleotide sequence encoding a first marker protein heterologous to the first cell line,
      (c) said first polynucleotide sequence encoding a first marker protein being flanked by site specific recombination sites ("SSRSs"), a 5' SSRS and a 3' SSRS; and
      (d) a second polynucleotide sequence encoding a second marker protein heterologous to the first cell line, being located 3' to the 3' SSRS;
   - providing a second genotype capable of expressing a recombinase being specific for said SSRSs;
   - crossing said first genotype with said second genotype wherein said recombinase deletes the first polynucleotide sequence encoding a first marker protein so as to obtain a third genotype wherein said promoter is operably linked to said second polynucleotide sequence encoding said second marker protein and wherein the recombinase gene is present in said third genotype;
   - crossing said third genotype with a fourth genotype capable of expressing a recombinase being specific for said SSRSs thereby obtaining a fifth genotype wherein said promoter is operably linked to said second polynucleotide sequence encoding said second marker protein and wherein the recombinase gene is not present in said fifth genotype;
   - crossing said fifth genotype with a sixth genotype, said sixth genotype containing a third polynucleotide comprising
      (a) a promoter operably linked to
      (b) a third polynucleotide sequence encoding a third marker protein heterologous to the first genotype,
      (c) said third polynucleotide sequence encoding a third marker protein being flanked by SSRSs, a 5' SSRS and a 3' SSRS; and
      (d) said second polynucleotide sequence encoding said second marker protein heterologous to the first genotype, being located 3' to the 3' SSRS;
   thereby obtaining a seventh genotype which is heterozygous and wherein on one allele said promoter is operably linked to said second polynucleotide sequence encoding said second marker protein and on the other allele said third polynucleotide is present; and
   - obtaining a double marker cell line, especially a double marker embryonic stem (ES) cell line, from said seventh genotype.
4. Method for producing a genotype capable of expressing a gene of interest, said gene of interest being heterologous for said genotype characterised in that the eighth genotype is introduced into the double marker ES cell line, according to claim 1, to obtain a ninth genotype,
   - said eighth genotype containing a fourth polynucleotide comprising a gene of interest encoding a gene to be expressed in said ninth genotype and being flanked by DNA sequences which are homologous to the 5' and 3' regions from the marker genes in said seventh genotype so as to allow homologous recombination between at least one allele of the seventh genotype and said eighth genotype;
   - said ninth genotype being heterozygous and comprising either
      (a) on one allele said second polynucleotide sequence encoding said second marker protein, being located 3' to the 3' SSRS, and on the other allele said fourth polynucleotide being flanked by SSRSs, a 5' SSRS and a 3' SSRS, is present ("ninth genotype A");
         or
      (b) on one allele said third polynucleotide sequence encoding said third marker protein being flanked by SSRS, a 5' SSRS and a 3' SSRS and on the other allele said fourth polynucleotide being flanked by SSRSs, a 5' SSRS and a 3' SSRS, is present ("ninth genotype B");
      said ninth genotype therefore being either (a) capable of expressing said gene of interest together with said second marker ("ninth genotype A") or (b) capable of expressing said gene of interest together with said third marker ("ninth genotype B").
5. Method according to embodiment 4, characterised in that said heterozygous ninth genotype is made homozygous by crossing or targeting steps thereby obtaining a homozygous ninth genotype.
6. Method according to embodiment 5, characterised in that a non-human animal is produced from said ninth genotype.
7. Method according to any one of embodiments 3 to 6, characterised in that said third marker is identical to said first marker.
8. Method according to any one of embodiments 3 to 7, characterised in that said SSRSs are selected from Cre-loxP sites, Flp-frt sites and Dre-Rox sites.
9. Method according to any one of embodiments 1 to 8, characterised in that each of said first, second, third and fourth polynucleotide, where present in such embodiment, comprise a gene encoding a selection marker, preferably under the control of an eukaryotic promoter, especially under the control of the murine Phosphoglucokinase gene (PGK) promoter.
10. Method according to embodiment 9, characterised in that said gene encoding said selection marker is flanked by SSRSs, said SSRSs being different from said SSRSs which flank said first, second or third marker, said gene encoding said selection marker is preferably flanked by Flp-recombinase target (frt) sites, a 5' frt site and a 3' frt.
11. Method according to any one of embodiments 1 to 10, characterised in that said genotypes and cell lines, especially ES cell lines, are murine, rat, rabbit, jack rabbit, primate, preferably human, cat, dog, sheep, goat, bovine, pig, frog, fish, insect, plant, especially moss, tobacco, maize, Arabidopsis, rice or barley, equine or guinea pig genotypes and cell lines,
   especially murine or rat genotypes and cell lines.
10. Method according to any one of embodiments 7 to 9, characterised in that said selection marker gene is an antibiotic resistance gene, preferably selected from neomycin, hygromycin, puromycin, 6-thioguanine and zeocin resistance genes.
13. Method according to any one of embodiments 1 to 12, characterised in that at least one of said first, second or third markers, where present in said embodiment, are fluorescence markers, preferably tandem dimer tomato (tdTomato), green fluorescent protein (GFP), enhanced green fluorescent protein (EGFP), red fluorescent protein (RFP), enhanced blue fluorescent protein (EBFP), enhanced cyan fluorescent protein (ECFP), yellow fluorescent protein (YFP), or orange fluorescent protein.
14. Method according to any one of embodiments 1 to 13, characterised in that at least one of said first, second or third markers, where present in such embodiment, are chromogenic, colour, luminescence or infrared markers, preferably selected from LacZ, GUS and luciferase.
15. Method according to any one of embodiments 1 to 14, characterised in that said first, second, third or fourth polynucleotide sequence, where present in such embodiment, is integrated at a predetermined locus in the genome of the cell, preferably at the Rosa26 locus, at the HPRT locus, at the beta-actin locus, at the Col1A1 locus, at an artificially introduced locus of a yeast artificial chromosome (YAC) or a bacterial artificial chromosome, especially at the Rosa26 locus.
16. Method according to any one of embodiments 1 to 15, characterised in that said first promoter is the CMV/beta-actin enhancer (CA) promoter (pCA).
17. Method according to any one of embodiments 1 to 16, characterised in that said ES cells are injected into blastocysts and implanted into an animal suitable for giving birth to embryos derived from such ES injected blastocysts to obtain animals having said seventh genotype and optionally breeding said animal having said seventh genotype with a wild type animal to further achieve homozygosity.
18. Double marker cell line, especially an embryonic stem cell line, obtainable by a method according to any one of embodiments 1 to 17.
19. Double marker cell line with a genotype which is heterozygous and wherein on one allele a promoter is operably linked to a polynucleotide sequence encoding a first marker protein and on the other allele a promoter is operably linked to a polynucleotide sequence encoding a second marker.
20. Double marker cell line according to embodiment 18 or 19, characterised in that said polynucleotide sequence encoding a first marker protein and said polynucleotide sequence encoding a second marker are integrated at a known and predetermined position the genome of the cell line.
21. Double marker cell line according to embodiment 18 to 20, characterised in that said promoters are identical and are the pCA promoter, the PGK promoter or the Rosa26 promoter.
22. Double marker cell line according to embodiment 18 to 21, characterised in that it comprises a heterologous gene for a selection marker, preferably neomycin or Hygromycin, under the control of a promoter, preferably the PGK promoter.
23. Double marker cell line according to any one of embodiments 18 to 22, characterised in that said polynucleotide sequence encoding a first marker or said polynucleotide sequence encoding a third marker and said polynucleotide sequence encoding a second marker are integrated at the Rosa26 locus.
24. Double marker cell line according to any one of embodiments 18 to 23, characterised in that said cell line is murine, rat, rabbit, jack rabbit, primate, preferably human, cat, dog, sheep, goat, bovine, equine or guinea pig, especially murine or rat.
25. Double marker cell line according to any one of embodiments 18 to 24, characterised in that it is an embryonic stem cell line.
26. Double marker cell line according to any one of embodiments 18 to 25, characterised in that at least one of said two markers, preferably both markers, are fluorescence markers, preferably tandem dimer tomato (tdTomato), green fluorescent protein (GFP), enhanced green fluorescent protein (EGFP), red fluorescent protein (RFP), enhanced blue fluorescent protein (EBFP), enhanced cyan fluorescent protein (ECFP), yellow fluorescent protein (YFP), or orange fluorescent protein, especially said two markers are tdTomato and EGFP.
27. Genotype obtainable according to any one of embodiments 4 to 17, being capable of expressing a gene of interest.

## Claims

1. A method for producing a double marker cell line comprising the following steps:
- providing a first genotype containing a first polynucleotide comprising
(a) a promoter operably linked to
(b) a first polynucleotide sequence encoding a first marker protein heterologous to the first cell line,
(c) said first polynucleotide sequence encoding a first marker protein being flanked by site specific recombination sites ("SSRSs"), a 5' SSRS and a 3' SSRS; and
(d) a second polynucleotide sequence encoding a second marker protein heterologous to the first cell line, being located 3' to the 3' SSRS;
- providing a second genotype capable of expressing a recombinase being specific for said SSRSs;
- crossing said first genotype with said second genotype wherein said recombinase deletes the first polynucleotide sequence encoding a first marker protein so as to obtain a third genotype wherein said promoter is operably linked to said second polynucleotide sequence encoding said second marker protein and wherein the recombinase gene is present in said third genotype;
- crossing said third genotype with a fourth genotype capable of expressing a recombinase being specific for said SSRSs thereby obtaining a fifth genotype wherein said promoter is operably linked to said second polynucleotide sequence encoding said second marker protein and wherein the recombinase gene is not present in said fifth genotype;
- crossing said fifth genotype with a sixth genotype, said sixth genotype containing a third polynucleotide comprising
(a) a promoter operably linked to
(b) a third polynucleotide sequence encoding a third marker protein heterologous to the first genotype,
(c) said third polynucleotide sequence encoding a third marker protein being flanked by SSRSs, a 5' SSRS and a 3' SSRS; and
(d) said second polynucleotide sequence encoding said second marker protein heterologous to the first genotype, being located 3' to the 3' SSRS;
thereby obtaining a seventh genotype which is heterozygous and
wherein on one allele said promoter is operably linked to said second polynucleotide sequence encoding said second marker protein and on the other allele said third polynucleotide is present; and
- obtaining a double marker cell line, especially a double marker embryonic stem (ES) cell line, from said seventh genotype.

2. Method for producing a genotype capable of expressing a gene of interest, said gene of interest being heterologous for said genotype **characterised in that** the eighth genotype is introduced into the double marker ES cell line, according to claim 1, to obtain a ninth genotype,
- said eighth genotype containing a fourth polynucleotide comprising a gene of interest encoding a gene to be expressed in said ninth genotype and being flanked by DNA sequences which are homologous to the 5' and 3' regions from the marker genes in said seventh genotype so as to allow homologous recombination between at least one allele of the seventh genotype and said eighth genotype;
said ninth genotype being heterozygous and comprising either
(a) on one allele said second polynucleotide sequence encoding said second marker protein, being located 3' to the 3' SSRS, and on the other allele said fourth polynucleotide being flanked by SSRSs, a 5' SSRS and a 3' SSRS, is present ("ninth genotype A"); or
(b) on one allele said third polynucleotide sequence encoding said third marker protein being flanked by SSRS, a 5' SSRS and a 3' SSRS and on the other allele said fourth polynucleotide being flanked by SSRSs, a 5' SSRS and a 3' SSRS, is present ("ninth genotype B");
said ninth genotype therefore being either (a) capable of expressing said gene of interest together with said second marker ("ninth genotype A") or (b) capable of expressing said gene of interest together with said third marker ("ninth genotype B").

3. Method according to claim 2, **characterised in that** said heterozygous ninth genotype is made homozygous by crossing or targeting steps thereby obtaining a homozygous ninth genotype.

4. Method according to claim 3, **characterised in that** a non-human animal is produced from said ninth genotype.

5. Method according to any one of claims 1 to 4, **characterised in that** said third marker is identical to said first marker.

6. Method according to any one of claims 1 to 5, **characterised in that** at least one of said first to fourth polynucleotide comprise a gene for a selection marker, preferably an antibiotic resistance gene, preferably selected from neomycin, hygromycin, puromycin, 6-thioguanine and zeocin resistance genes.

7. Method according to any one of claims 1 to 6, **characterised in that** at least one of said first, second or third markers are fluorescence markers, preferably tandem dimer tomato (tdTomato), green fluorescent protein (GFP), enhanced green fluorescent protein (EGFP), red fluorescent protein (RFP), enhanced blue fluorescent protein (EBFP), enhanced cyan fluorescent protein (ECFP), yellow fluorescent protein (YFP), or orange fluorescent protein.

8. Method according to any one of claims 1 to 7, **characterised in that** said first, second, third or fourth polynucleotide sequence is integrated at a predetermined locus in the genome of the cell, preferably at the Rosa26 locus, at the HPRT locus, at the beta-actin locus, at the Col1A1 locus, at an artificially introduced locus of a yeast artificial chromosome (YAC) or a bacterial artificial chromosome, especially at the Rosa26 locus.

9. Method according to any one of claims 1 to 8, **characterised in that** said ES cells are injected into blastocysts and implanted into an animal suitable for giving birth to embryos derived from such ES injected blastocysts to obtain animals having said seventh genotype and optionally breeding said animal having said seventh genotype with a wild type animal to further achieve homozygosity.

10. Double marker cell line, especially an embryonic stem cell line, obtainable by a method according to any one of claims 1 and 3 to 9.

11. Double marker cell line with a genotype which is heterozygous and wherein on one allele a promoter is operably linked to a polynucleotide sequence encoding a first marker protein and on the other allele a promoter is operably linked to a polynucleotide sequence encoding a second marker.

12. Double marker cell line according to claim 10 or 11, **characterised in that** said polynucleotide sequence encoding a first marker protein and said polynucleotide sequence encoding a second marker are integrated at a known and predetermined position the genome of the cell line, especially at the Rosa26 locus.

13. Double marker cell line according to claim 10 to 12, **characterised in that** it comprises a heterologous gene for a selection marker, preferably neomycin or Hygromycin, under the control of a promoter, preferably the PGK promoter.

14. Double marker cell line according to any one of claims 10 to 13, **characterised in that** it is an embryonic stem cell line.

15. Genotype obtainable according to any one of claims 1 to 9, being capable of expressing a gene of interest.
